(19) Europäisches Patentamt
European Patent Office
Office européen des brevets

(11) Publication number: 0 120 585
B1

(12) **EUROPEAN PATENT SPECIFICATION**

(45) Date of publication of patent specification: **16.12.87**

(21) Application number: **84301026.5**

(22) Date of filing: **17.02.84**

(51) Int. Cl.⁴: **C 07 D 513/04,**
A 61 K 31/425, A 61 K 31/445
// (C07D513/04, 333:00, 275:00)

# (54) Fused heterocyclic compounds.

(30) Priority: **22.02.83 US 468221**
**28.04.83 GB 8311653**

(43) Date of publication of application:
**03.10.84 Bulletin 84/40**

(45) Publication of the grant of the patent:
**16.12.87 Bulletin 87/51**

(84) Designated Contracting States:
**AT BE CH DE FR IT LI LU NL SE**

(58) References cited:
**DE-A-2 534 689**
**GB-A-2 007 227**
**GB-A-2 023 133**
**US-A-4 128 658**
**US-A-4 165 378**

**Chemical Abstracts, vol. 96, no. 15, 12 April 1982, Columbus, Ohio (US); K.G.JENSEN et al.: "Phosphoramides. XIX. Phosphorus pentoxide amine hydrochloride reagents in the synthesis of 3-amino-1,2-benzisothiazole 1,1-dioxides and 3-aminothieno3,4-drisothiazole 1,1-dioxides", p. 689, column 2, abstract 122677d**

(73) Proprietor: **AMERICAN HOME PRODUCTS CORPORATION**
**685, Third Avenue**
**New York, New York 10017 (US)**

(72) Inventor: **Schiehser, Guy Alan**
**914 Charleston Greene**
**Malvern Pennsylvania 19355 (US)**
Inventor: **Strike, Donald Peter**
**445 Ivan Avenue**
**St. Davids Pennsylvania 19087 (US)**

(74) Representative: **Wileman, David Francis et al**
**c/o John Wyeth and Brother Limited**
**Huntercombe Lane South**
**Taplow Maidenhead Berkshire SL6 OPH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 120 585 B1

**Description**

This invention relates to new fused heterocyclic compounds, more particularly to fused heterocyclic compounds having a selective action on $H_2$ histamine receptors and which inhibit gastric acid secretion, to processes for their preparation and to pharmaceutical compositions containing them.

It has been postulated that the physiologically active compound histamine, which occurs naturally within the animal body, is able to combine, in the course of exerting its activity, with certain specific receptors of which there are at least two distinct and separate types. The first has been named the $H_1$ receptor (Ash and Schild, Brit. J. Pharmac., 1966, 27, 427); and the action of histamine at this receptor is blocked (antagonized) by classical "antihistamine" drugs such as mepyramine (pyrilamine). The second histamine receptor has been named the $H_2$ receptor (Black et al., *Nature*, 1972, 236, 385) and the action of histamine at this receptor is blocked by drugs such as cimetidine. It is known that one of the results of the blockage of the action of histamine at the $H_2$ receptor is the inhibition of the secretion of gastric acid and a compound which possesses this ability is therefore useful in the treatment of peptic ulcers and other conditions caused or exacerbated by gastric acidity, including stress ulcers and gastrointestinal bleeding due to trauma.

The commercialization of cimetidine and subsequent follow-up pharmacological research in patients has demonstrated that cimetidine is a drug with limitations, such as short duration of action, anti-androgenic activity, and a tendency to cause confusional states in elderly patients. Obviously, much intensive research has been carried out to find improved $H_2$ antagonists. Indeed, selective $H_2$ antagonists having greater activity than cimetidine have been discovered. Among the better known new $H_2$ antagonists are ranitidine (disclosed in U.S. Patent No. 4,128,658) having the structure:

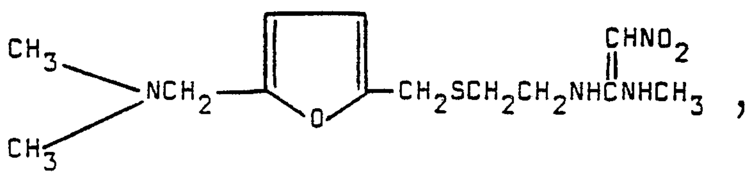

tiotidine (U.S. Patent No. 4,165,378) having the structure:

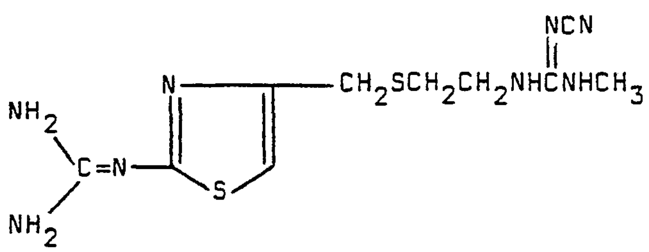

and compounds such as those disclosed in European Patent Application 24,510 having the structure:

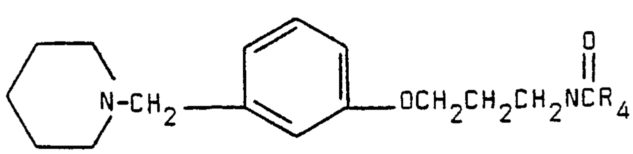

wherein $R_4$ is among others, hydrogen, methyl or methylol and those disclosed in U.K. Patent No. 2,067,987 having the structure:

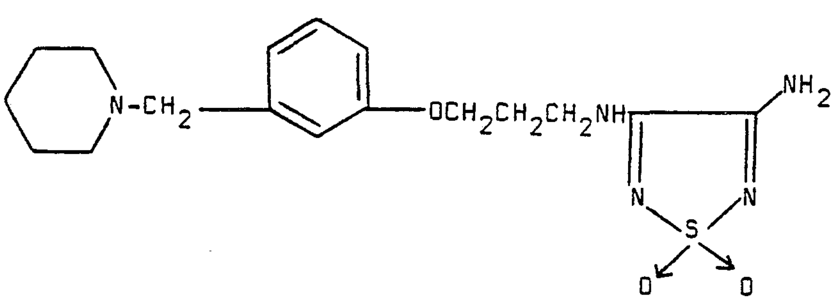

The present invention provides a novel group of compounds, with potent $H_2$ receptor antagonist activity, having the following formula:

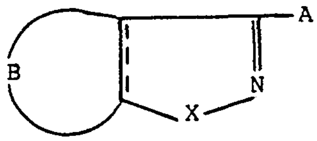

(I)

wherein B is a moiety having the formula:

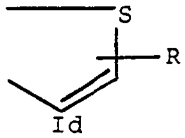

Id

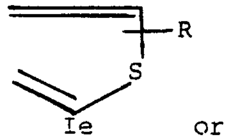

Ie

or

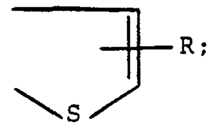

If

the dotted line represents a bond which is present when B is formula Id or If and absent when B is formula Ie, R is hydrogen, mono- or dihalo, amino, nitro, cyano, hydroxy, trifluoromethyl, mercapto, lower alkyl, lower alkoxy, alkanoyl of 2 to 5 carbon atoms, cycloalkyl of 5 to 7 carbon atoms, alkoxycarbonyl, mono- or di-lower alkyl substituted amino, alkanoylamino of 2 to 5 carbon atoms, lower alkyl thio, loweralkylsulfonyl, sulfamoyl, lower alkyl substituted sulfamoyl, phenyl or phenyl substituted with halo, lower alkyl, lower alkoxy, trifluoromethyl, hydroxy, amino, cyano or nitro; X is $SO_2$, SO, S or C=O and A is an amine of formula:—

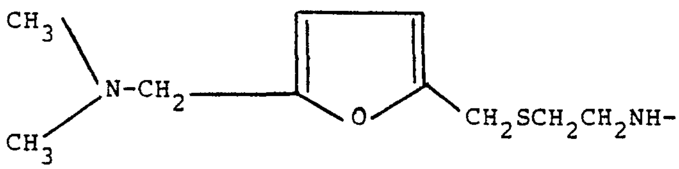

(Ia),

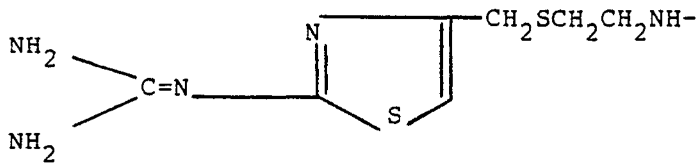

(Ib)

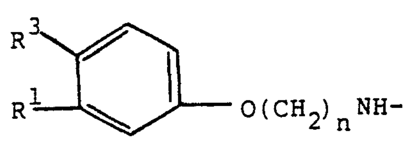

(Ic)

wherein n is 1 to 4; $R^1$ is hydrogen or $R^2CH_2$ wherein $R^2$ is mono- or diloweralkylamino, mono- or di-N-loweralkylamino lower alkyl, (2-furyl)methylamino, benzylamino, lower cycloalkylamino or 5 to 7 carbon atoms, 1-pyrrolidinyl, 1-piperidinyl, 1-hexahydroazepinyl, 1-octahydroazocinyl, 3-thiazolidinyl, 4-morpholinyl or 4-thiomorpholinyl; $R^3$ is hydrogen or (1-piperidinyl)methyl with the proviso that when $R^3$ is (1-piperidinyl)methyl, then $R^1$ is hydrogen; and the pharmacologically acceptable salts thereof.

The term "halo" refers to fluoro, chloro and bromo. The terms "lower alkyl" and "lower alkoxy" refer to moieties having 1—6 carbon atoms in the carbon chain. The term "alkanoyl" refers to the moiety $R^aCO$— wherein $R^a$ is an alkyl group having 1—4 carbon atoms. The term "cycloalkyl" refers to cyclic structures having 5 to 7 carbon atoms.

Preferred values are:

(i) A has formula Ic or Ia,

(ii) B has formula Ie,

(iii) $R^3$ is hydrogen,

(iv) n is 3,

(v) $R^1$ is $R^2CH_2$ where $R^2$ is 1-piperidinyl, and/or

(vi) X is $SO_2$.

In general the compounds of formula I are prepared by building up the molecule from appropriate reactive precursors. For example the compounds of the invention can be readily prepared by reacting a 3-alkylthio derivative of an appropriate thienoisothiazole starting material with the desired amine according to the following reaction sequence:—

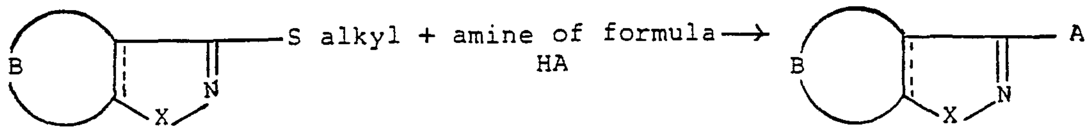


where the dotted line, B, R, X and A are as hereinbefore defined and hal represents a halogen. Alternatively "S-alkyl" may be replaced by a -S-aralkyl group. The thieno starting materials in the sequence above can be prepared according to the following reaction sequence exemplified for 3-(methylthio)thieno-[3,4-*d*]isothiazole-1,1-dioxide:

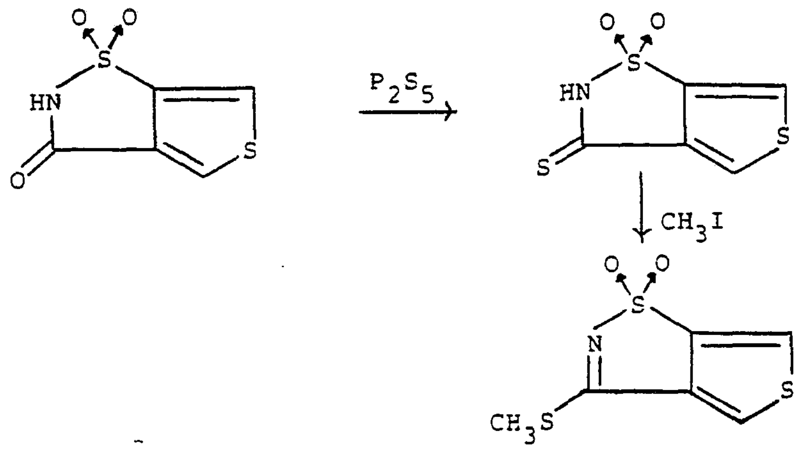


The starting compound thieno[3,4-*d*]isothiazol-3(2*H*)-one-1,1-dioxide can be prepared according to the procedure of P. A. Rossy et al., J. Org. Chem., *45* 617 (1980). Routes to 3-oxo thienoisothiazoles are also disclosed in U.S. Patent 4,028,373. The amines of formula HA are well known in the field of $H_2$-receptor antagonists and their preparation is reported in the following patent literature:

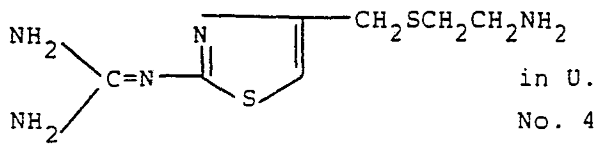

in U.S. Patent No. 4,165,378;

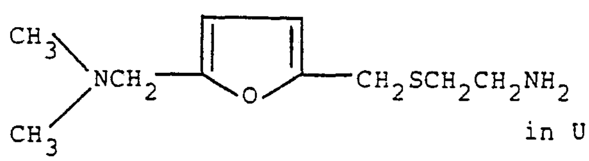

in U.S. Patent No. 4,128,658; and

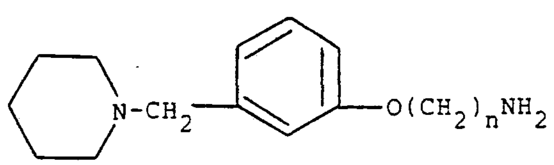

in U.K. Patent No. 2,023,133.

The amines having the formula

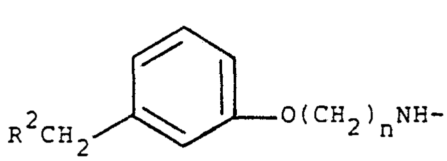

or

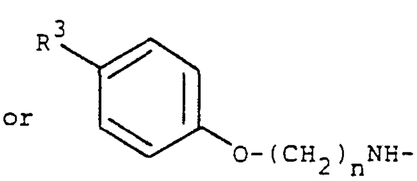

wherein $R^2$, $R^3$ and n are as defined hereinbefore, can be prepared according to the following reaction sequence (exemplified for the case where $R^3$ is hydrogen):

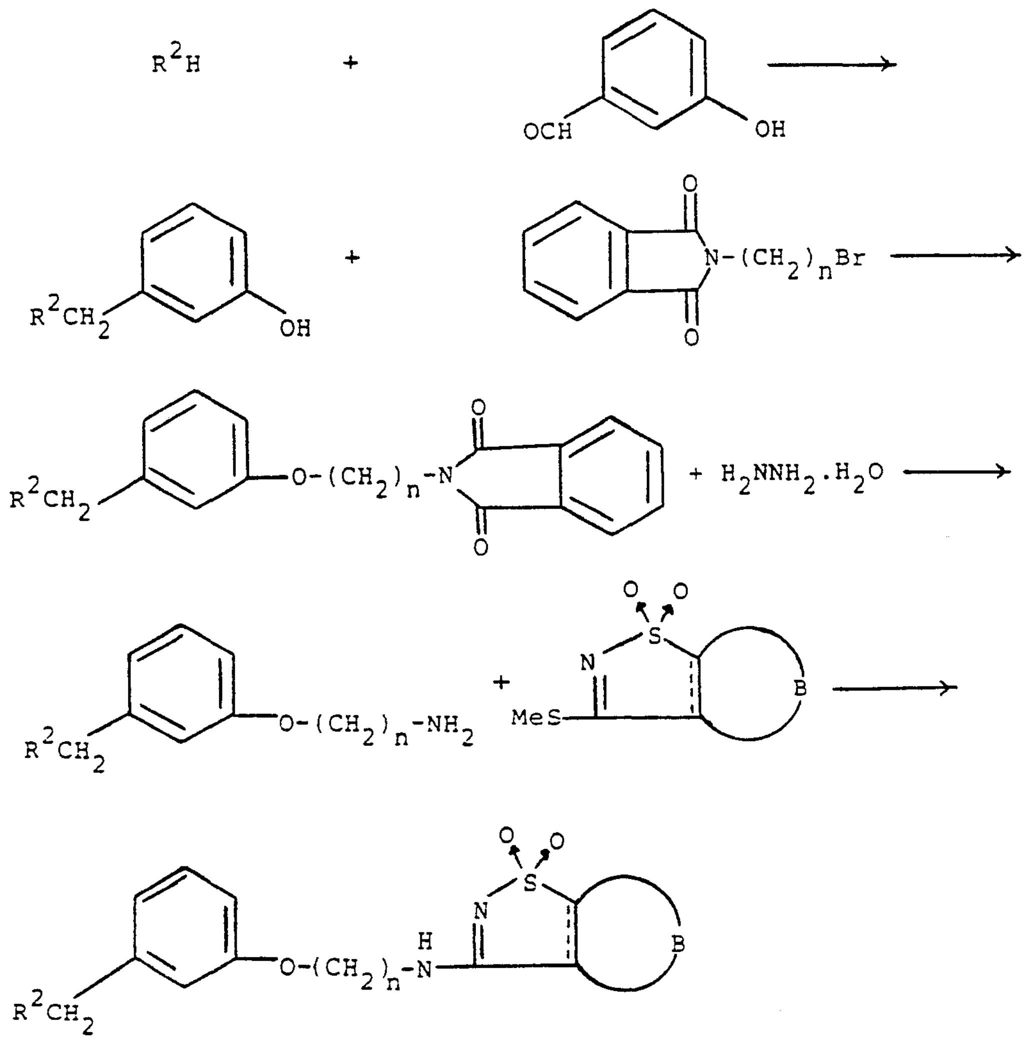

As an alternative procedure, the preparation of the isoindoledione reactant can be carried out as follows:

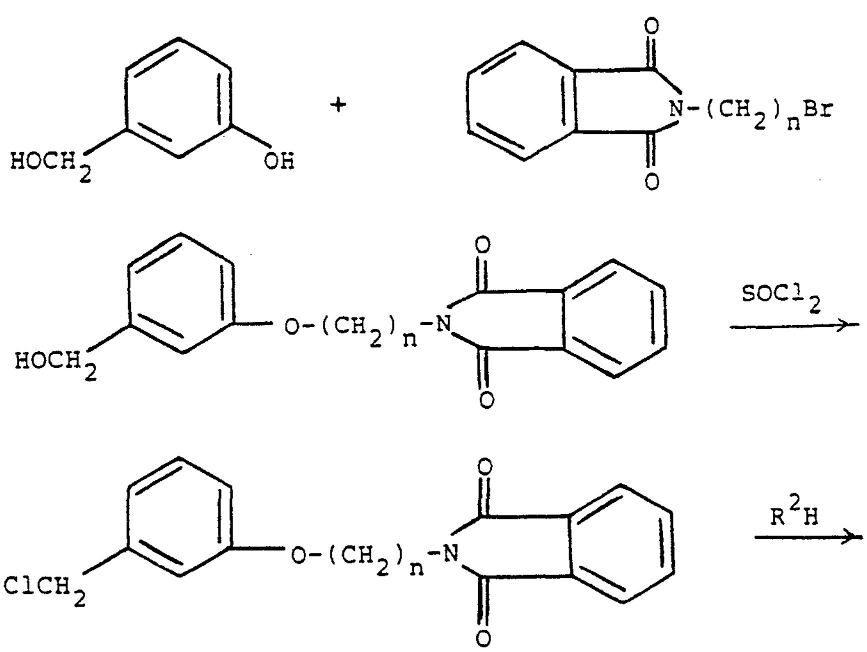

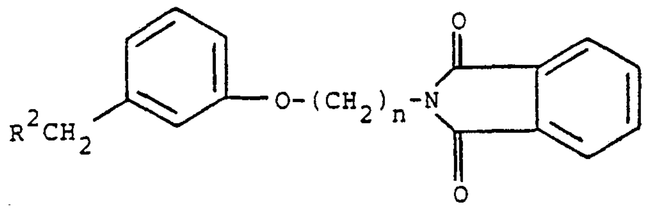

Accordingly this invention provides a process for preparing a compound of formula I as hereinbefore defined which comprises:

a) reacting a compound of formula

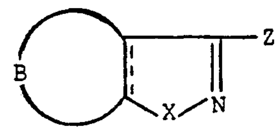

(II)

wherein the dotted line, X and B are as hereinbefore defined and Z is $SR^4$ where $R^4$ is alkyl or aralkyl especially alkyl of 1 to 6 carbon atoms or aralkyl of 7 to 10 carbon atoms, e.g. benzyl, with an amine of formula H—A wherein A is as hereinbefore defined to give a compound of formula I, or

b) reacting a compound of formula

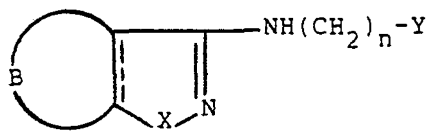

(III)

wherein the dotted line, X, B and n are as hereinbefore defined and Y is OH or L where L represents a leaving group such as halogen or an equivalent replaceable group such as a sulphonyloxy radical e.g. alkyl or aryl sulphonyloxy, especially tosyloxy;
with a compound of formula:

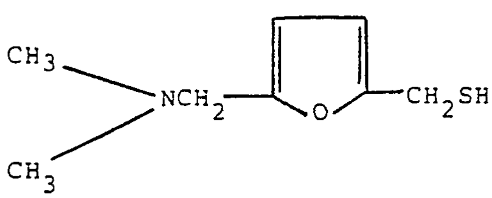

(IVa)

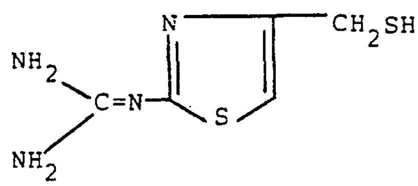

(IVb)

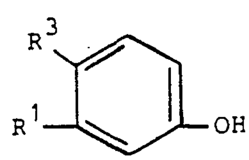

(IVc)

wherein $R^1$ and $R^3$ are as hereinbefore defined, with the proviso that (i) when a compound of formula IVa or IVb is used then n is 2 in the compound of formula III and (ii) when a compound of formula IVc is used then Y is L;

c) reacting a compound of formula

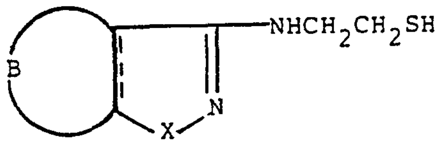

(V)

wherein the dotted line, B and X are as defined above, with a compound of formula:

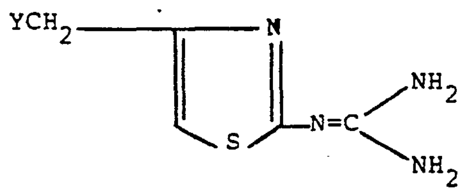

(VIa)

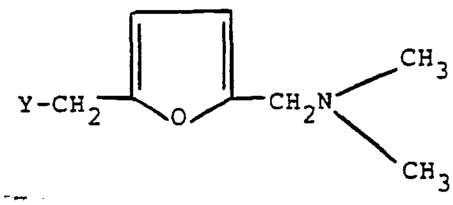

(VIb)

wherein Y is OH or L as defined above to give a compound of formula I wherein A has the formula Ia or Ib;

d) reacting an aldehyde of formula

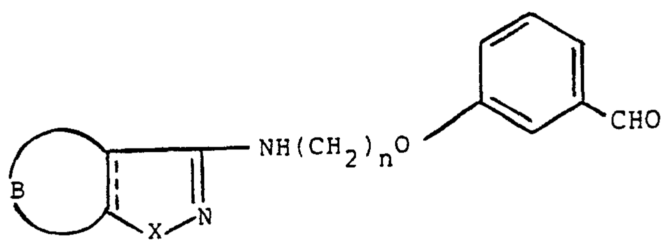

(VIIa)

with an appropriate amine of formula

$HR^2$ (VIIb)

in which formulae the dotted line, n, B, X and $R^2$ are as defined above, under reductive amination conditions to give a corresponding compound of formula I wherein $R^1$ is $R^2CH_2$— and $R^3$ is hydrogen; or

e) reacting an aldehyde of formula

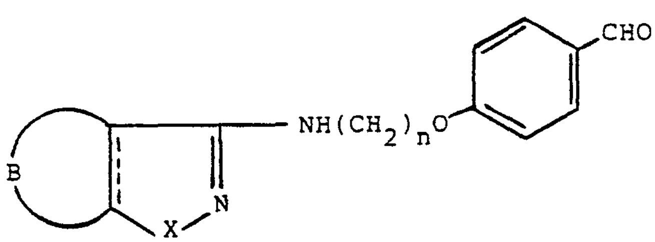

(VIIc)

wherein the dotted line, n, B and X are as hereinbefore defined with piperidine under reductive amination conditions to give a compound of formula I wherein $R^3$ is (1-piperidinyl)-methyl; or

f) reacting an aldehyde of formula

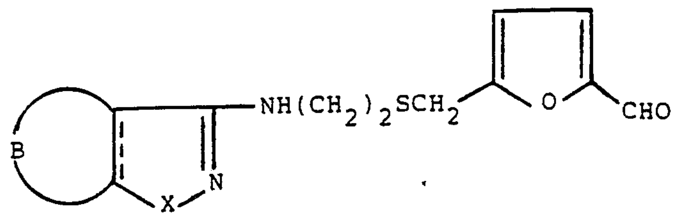

(VIId)

wherein the dotted line, B and X are as hereinbefore defined with dimethylamine under reductive amination conditions to give a compound of formula I wherein A has formula Ia; or

g) reacting a compound of formula

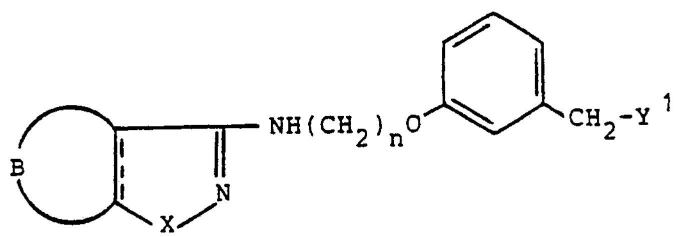

(VIII)

wherein the dotted line, n, X and B are as hereinbefore defined and $Y^1$ is OH or L wherein L is a leaving group such as hereinbefore described, with an amine of formula $HR^2$ as defined above, the reaction being carried out in the presence of a nickel catalyst when $Y^1$ is OH, to give a compound of formula I wherein A has the formula Ic and $R^1$ is $R^2CH_2$—; or

h) reacting a compound of formula

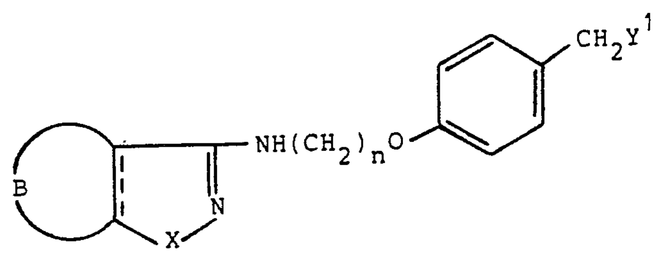

(VIIIa)

wherein the dotted line, $Y^1$, n, X and B are as hereinbefore defined, with piperidine, the reaction being carried out in the presence of a nickel catalyst when $Y^1$ is OH, to give a compound of formula I wherein A has the formula Ic and $R^3$ is (1-piperidinyl)methyl; or

i) reacting a compound of formula

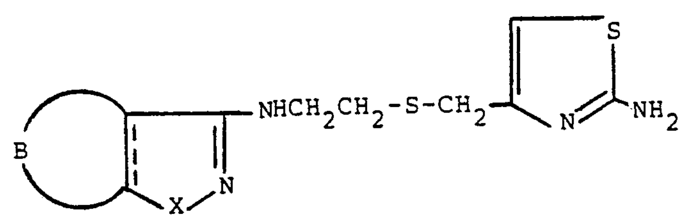

(IX)

wherein the dotted line, X and B are as hereinbefore defined with a compound capable of converting —$NH_2$ to

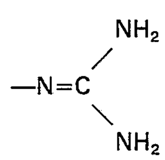

to give a compound of formula I wherein A has formula Ic,

j) reacting a compound of formula

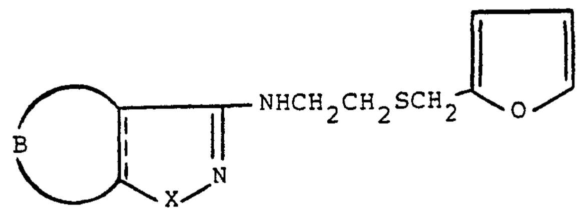

(X)

wherein the dotted line, X and B are as hereinbefore defined with dimethylamine in the presence of formaldehyde or paraformaldehyde, to give a compound of formula I wherein A has formula Ia, or

k) reacting a compound of formula

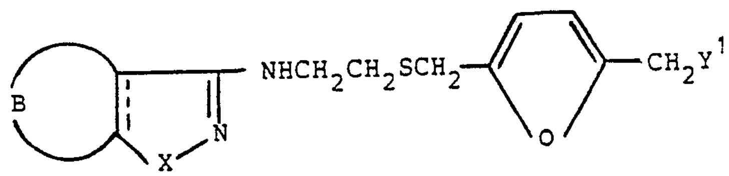

(XI)

wherein $Y^1$ is OH or a leaving group, the dotted line, L and B are as hereinbefore described, with dimethylamine; the reaction being carried out in the presence of a nickel catalyst when $Y^1$ is OH; or

l) a compound of formula I having a reactive substituent group is converted to a compound of formula I having a different substituent group; or

m) a basic compound of formula I is converted to a pharmacologically acceptable salt or a pharmacologically acceptable salt is converted to the free base form.

Processes b) and c) may be carried out in an inert solvent such as an alkanol, acetonitrile, dimethylformamide, dimethylsulfoxide, acetone and the like; if desired with heating. Preferably the reaction is conducted in the presence of base which acts as acid acceptor. Suitable bases include for example NaOH, KOH, LiOH, triethylamine, dimehtylaniline, sodium ethoxide and the like. When Y is OH in the compounds of formulae III or VI or VIb then the reaction is carried out in the presence of a mineral acid e.g. concentrated HCl.

Processes d), e) and f) may be carried out using a hydride transfer agent to effect reductive amination, e.g. an alkali metal borohydride such as sodium borohydride, and in an inert solvent, e.g. an alkanol such as ethanol.

When $Y^1$ is a leaving group in the compound of formula VIII, VIIIa or XI then processes g), h) and k) may be carried out in an inert solvent, e.g. acetonitrile, dimethylformamide preferably in the presence of an acid acceptor, e.g. triethylamine or $K_2CO_3$.

When $Y^1$ is OH in the compounds of formulae VIII, VIIIa or XI then processes g), k) and h) may be carried out in the presence of a nickel catalyst such as Raney nickel e.g. Raney nickel W2. An organic solvent which is inert under the reaction conditions, is usually used, for example xylene, toluene or benzene. Preferably the reaction is carried out by heating the reactants under reflux in a water immiscible organic solvent, e.g. xylene, and removing the water formed during reaction by azeotropic distillation. If necessary reactive substituent groups can be blocked during reaction and released later.

Process i) is carried out by standard procedures known in the art for converting an amine function to a guanidine function, e.g. using urea, thiourea, cyanamide or a reagent of formula

$NH_2$ C—$L^1$
||
NH

where $L^1$ is a halogen, S-alkyl or S-alkoxy.

For example the reaction with an S-alkylisothiourea may be effected by heating the reactants in a suitable solvent, e.g. acetonitrile or an alkanol.

Process j) may be carried out using standard procedures well known in the art for effecting the Mannich reaction.

Many of the intermediates used in the reactions above are known compounds or are prepared by analogous methods for known compounds. Compounds of formula III can be prepared by reacting a compound of formula II with a corresponding amine of formula

$$NH_2(CH_2)_n—Y \qquad (XII)$$

Compounds of formula IVa and IVb may be prepared by treating the corresponding chloromethyl analogues with NaSH. Similarly compounds of formula V may be prepared by reacting corresponding

compounds of formula III where Y is halogen, e.g. chlorine, with NaSH.

Compounds of formula VIa and VIb wherein Y is a leaving group may be prepared for example from the corresponding alcohols by conventional means.

Compounds of formula VIIa, VIIc and VIId may be prepared by reacting the appropriate compound of formula III wherein Y is chloro with 3- or 4-hydroxybenzaldehyde or 5-mercapto methylfuran-2-carboxaldehyde in the manner of process b).

Compounds of formulae VIII, VIIIa and XI may be prepared by reacting a compound of formula II with the appropriate amine starting materials wherein $Y^1$ is OH in the manner of process a) and if desired converting the hydroxy $Y^1$ group to a leaving group by conventional means.

Similarly compounds of formulae IXC and X may be prepared by reacting a compound of formula II with an appropriate compound of formula

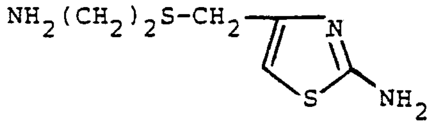 or 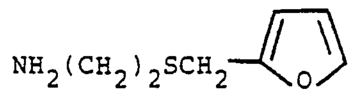

in the manner of process a).

Once a compound of formula I having a reactive substituent group has been prepared then that compound may be converted in known manner to other compounds of formula I. For example when R is a lower alkoxy substituent dealkylation produces a corresponding compound of formula I wherein R is a hydroxy substituent. When R is nitro then reduction, e.g. using ammonium sulphide can convert the nitro group to an amino group. Such amino groups may be acylated.

In any of the aforementioned processes reactive substituent groups may be blocked and released at a later stage.

The compounds of the invention readily form pharmacologically acceptable salts with both inorganic and organic acids, such as hydrochloric, hydrobromic, sulfuric, nitric, phosphoric, acetic, maleic, fumaric, citric and methanesulfonic.

The compounds of the invention have histamine $H_2$-blocking activity and can be used in the treatment of conditions where there is hypersecretion of gastric acid, such as in gastric and peptic ulceration, and other conditions caused or exacerbated by gastric acidity such as stress ulceration or gastric intestinal bleeding due to trauma.

The compounds of the invention can be administered orally or parenterally or by suppository, of which the preferred route is the oral route. They may be used in the form of the base or as a pharmacologically acceptable salt. They will in general be associated with a pharmaceutically acceptable carrier or diluent, to provide a pharmaceutical composition.

Accordingly this invention also provides a pharmaceutical composition comprising a compound of formula I or a pharmacologically acceptable salt thereof and a pharmaceutically acceptable carrier.

The compounds of the invention can be administered in combination with other active ingredients, e.g. conventional antihistamines if required. For oral administration the pharmaceutical composition can most conveniently be in the form of capsules or tablets, which may be slow release tablets. The composition may also take the form of a dragee or may be in syrup form.

A convenient daily dose by the oral route would be of the order of 100 mg to 1.2 g per day, in the form or dosage units containing from 20 to 200 mg per dosage unit. A convenient regimen in the case of a slow release tablet would be twice or three times a day.

Parenteral administration may be by injections at intervals or as a continuous infusion. Injection solutions may contain from 10 to 100 mg/ml of active ingredient.

The histamine $H_2$-antagonist activity of the compounds of the invention may be demonstrated by the ability of the compounds to inhibit the histamine-induced positive chronotropic response in the spontaneously beating right atrium of the guinea pig heart, as well as by activity in other more generalised procedures, such as the modified Shay procedure of pylorus ligation for the study of rat gastric secretion and by the study of gastric secretion in the unanaesthetised dog. The procedures for these tests and the results for some of the compounds of the invention are presented in the Examples (vide infra). The following illustrates the preparation of starting materials.

Preparation A

Preparation of 3-(Methylthio)Thieno[3,4-d]Isothiazole-1,1-Dioxide

A. Thieno[3,4-d]Isothiazol-3(2H)-Thione-1,1-Dioxide

To a mixture of 5.6 g (0.03 mole) of thieno[3,4-*d*]isothiazol-3(2*H*)-one-1,1-dioxide in 50 ml of dry pyridine is added 5.6 g (0.016 mole) of phosphorus pentasulphide portionwise over 3 minutes. The viscous mixture is slowly heated in an oil bath under an atmosphere of nitrogen.. The temperature of the oil bath is slowly increased to 80°C after 30 minutes. The temperature of the oil bath is then kept at 80°C for 25 minutes, the internal temperature reading 63°C. The solution is cooled to 50°C and is added dropwise over 5 minutes to 200 ml of water and cooled in an ice bath. The precipitate which forms is collected and discarded. The filtrate is cooled in ice and acidified with concentrated hydrochloric acid to pH 1. The

precipitate which forms is collected to yield 40% of material. In another experiment, a sample is recrystallised from water to obtain an analytical sample, m.p. 196—8°C (dec.).

*Analysis for:* $C_5H_3NO_2S_3$
*Calculated:* C, 29.26; H, 1.47; N, 6.82
*Found:* C, 29.91; H, 1.43; N, 6.87

B. 3-(Methylthio)Thieno[3,4-d]Isothiazole-1,1-Dioxide

To a mixture of 0.9 g (0.0044 mole) of thieno[3,4-*d*]isothiazol-3(2*H*)-thione-1,1-dioxide in 4 ml of ethanol is added a solution of 0.35 g (0.0044 mole) of 50% sodium hydroxide in 3 ml of water. To this thick mixture is added 0.62 g (0.0044 mole) of iodomethane. The mixture is heated under reflux for 5 minutes, and then filtered to give 0.35 g of product. On cooling, a second crop of 0.1 g of material is obtained. A small amount of the first crop is recrystallised from ethanol to afford an analytical sample, m.p. 184—6°.

*Analysis for:* $C_6H_5NO_2S_3$
*Calculated:* C, 32.86; H, 2.30; N, 6.39
*Found:* C, 32.76; H, 2.27; N, 6.43

The following examples illustrate the invention.

Example 1

N-[2-[[[5-[(Dimethylamino)Methyl]-2-Furanyl]Methyl]Thio]Ethyl]Thieno[3,4-d]Isothiazol-3-Amine-1,1-Dioxide

To a suspension of 1.8 g (0.0082 m) of 3-(methylthio)thieno[3,4-*d*]isothiazole-1,1-dioxide in 10 ml of ethanol is added 1.76 g (0.0082 m) of 2-[[[5-[(dimethylamino)methyl]-2-furanyl]methyl]thio]ethylamine with stirring at room temperature. The reaction mixture is heated to reflux and heating is continued for 1.5 hours. At this time the reaction mixture is filtered to remove insoluble material and then evaporated to dryness. The product is purified using a silica gel column with methanol as the eluant. The desired fraction was evaporated to dryness and then triturated with ether to give 120 mg a white solid with a m.p. 100—103°C.

*Analysis for:* $C_{15}H_{19}N_3O_3S_3$
*Calculated:* C, 46.19; H, 5.02; N, 10.77
*Found:* C, 46.05; H, 4.87; N, 11.04

Example 2

[4-[[[2-[(Thieno[3,4-*d*]Isothiazol-3-yl)Amino]Ethyl]Thio]Methyl]-2-Thiazolyl]Guanidine-S',S'-Dioxide

A mixture of 0.5 g (0.0022 mole) of [4-[[(2-aminoethyl)thio]methyl]-2-thiazolyl]guanidine and 0.48 g (0.0022 mole) of 3-(methylthio)thieno[3,4-*d*]isothiazole-1,1-dioxide is slowly heated in an oil bath. After the temperature of the oil bath reaches 130°C, the heating is terminated. When the temperature of the oil bath reaches 90°C, 25 ml of ethanol is added to the mixture. The mixture is then heated under reflux for 30 minutes, and then filtered. The filtrate, on standing, deposits a second crop, which is combined with the original filter cake. This combined filtered cake is dissolved in 300 ml of boiling ethanol. The solution is cooled to room temperature and diluted with petroleum ether to the cloudy point (about 500 ml). The mixture is cooled in ice to precipitate 0.5 g of product, m.p. 223—5° (dec.).

*Analysis for:* $C_{12}H_{14}N_6O_2S_4$
*Calculated:* C, 35.80; H, 3.51; N, 20.88
*Found:* C, 35.82; H, 3.68; N, 20.45

Example 3

*N*-[3-[3-(1-Piperidinylmethyl)Phenoxy]Propyl]Thieno[3,4-d]Isothiazol-3-Amine, 1,1-Dioxide

To a solution of 8.3 g (.038 moles) of 3-(methylthiothieno[3,4-*d*]isothiazole-1,1-dioxide in 20 ml of ethanol is added 9.4 g (.038 moles) of 3-[3-[(1-piperidinyl)methyl]phenoxy]propylamine in 20 ml of ethanol and this mixture is heated at reflux for 2 hours. Upon cooling, the product precipitates out and the crude material is then recrystallized from ethyl acetate (and treated with charcoal at the same time) to give an 8.0 g yield of product having a m.p. 145—7°C.

*Analysis for:* $C_{20}H_{25}N_3O_3S_2$
*Calculated:* C, 57.25; H, 6.01; N, 10.02
*Found:* C, 57.55; H, 6.18; N, 9.94

Example 4

The guinea pig heart atrium test is carried out as follows:

A guinea pig right atrium is suspended at 1 g tension (isometric) in a thermostatically controlled (32°C) tissue bath (10 ml) containing oxygenated (95% $O_2$; 5% $CO_2$) Krebs-Haenseleit buffer (pH 7.4). The tissue is allowed to stabilize over 1 hour. Individual contractions are recorded with a force-displacement transducer through a strain gauge coupler. A control dose-response curve to histamine in the above described tissue bath is obtained after which the tissue is washed three times and allowed to re-equilibrate to basal rate. The test compound is added to the tissue bath at the desired final concentration. Thirty minutes after addition of

the compound, a fresh histamine dose response curve is again obtained. Then the response to hist'amine in the presence of antagonist is compared to the histamine control response. This procedure is repeated, using fresh tissues, for each concentration of antagonist tested. The result is expressed as the apparent dissociation constant ($pA_2$) of the $H_2$ antagonist as determined by standard procedures. Cimetidine is used as the standard for this test.

The results for a series of compounds of the invention are as follows:

| Compound or Example No. | $pA_2$ Value |
|---|---|
| Cimetidine | 6.5 |
| 1 | $1.0 \times 10^{-9}$M* |
| 2 | $1.0 \times 10^{-7}$M* |
| 3 | $5.94 \times 10^{-9}$M* |

*These values are the $K_B$ values for the compounds tested, and the $K_B$ differs from the $A_2$ value only by the fact that the $A_2$ value reflects the results of three experiments, while the $K_B$ value represents the result of only one experiment.

The results show that the compounds of the invention are extremely active $H_2$ antagonists, being significantly more active than the standard compound cimetidine.

Example 5

The procedure for testing gastric secretion in the rat, a modification of the procedure of Shay et al, *Gastroenterology,* 26, 906—13 (1954) is carried out as follows:

Male Charles River rats weighing 200—300 grams are deprived of food but not water for 24 hours prior to use. Water is, however, witheld during the experiment. The rats are weighed, anesthetised, and the pylorus ligated according to the method of Shay et al. Treatment or vehicle control is then administered interduodenally (i.d.). Rats are housed 2/cage and sacrificed with $CO_2$ four hours after ligation. The stomachs are removed, rinsed, and contents emptied into a graduated centrifuge tube. The tubes are centrifuged for 20 minutes at 2,000 RPM and the volume of gastric juice recorded. Any samples obviously contaminated by feces, food or hemolysis are eliminated. An aliquot of each is frozen for later analysis of $Na^+$, $K^+$ and $Cl^-$ concentration. The pH is measured and 1 ml of gastric juice is titrated with 0.1N NaOH to a pH of 7.0—7.4. Titratable acid output is calculated in microequivalents and the percent inhibition of acid output is calculated as follows:

$$\%\ \text{Inhibition of Acid Output} = \frac{\underset{(\text{control})}{\text{Acid Output}} \quad \underset{(\text{Drug})}{\text{Acid Output}}}{\underset{(\text{control})}{\text{Acid Output}}} \times 100$$

The test results for some of the compounds of the invention and for the known $H_2$ antagonists ranitidine and tiotidine are as follows:

| Compound of Example No. | Dose (mg/kg) | % Inhibition |
|---|---|---|
| 1 | 16 | 87 |
| 3 | 8 | 91 |
| | 4 | 87 |
| | 1 | 84 |
| | 0.5 | 37 |
| ranitidine | 4 | 43 |
| tiotidine | 4 | 16 |

The results show the compounds of the invention to have significant activity in inhibiting gastric acid secretion.

Example 6

The procedure for testing the ability of the compounds of the invention to inhibit the secretion of acidic gastric juice in dogs is as follows:

A female pure bred beagle (7—10 kg) having Pavlov gastric pouches is fasted overnight with water *ad lib.* The animal is orally dosed and thirty minutes later it is fed to induce gastric acid secretions. Gastric acid samples are then collected every 15 minutes. The volume of each sample is measured and aliquots are titrated to neutrality with 0.1 N NaOH to determine acid concentration. The results are reported as the dose at which there is obtained a 50% inhibition of the total gastric acid output ($ID_{50}$).

The results for some compounds of the invention and the known $H_2$ antagonists cimetidine and ranitidine are presented below:

| Compound of Example No. | $ID_{50}$ (mg/kg) |
| --- | --- |
| 3* | 0.25 |
| cimetidine | 6.0 |
| ranitidine | 2.3 |

*This compound reduced gastric acid secretions in the dog by 82% at a dose of 1 mg/kg.

The results show that the compounds of the invention are extremely active in reducing gastric acid secretions in the dog at very low dosage levels, and that the levels are below the levels at which the same reduction in gastric acid secretion is attained by the known $H_2$ antagonists cimetidine and ranitidine.

Example 7

N-[2-[[[5-[(dimethylamino)methyl]-2-furanyl]methyl]thio]ethyl]-thieno[3,4-d]isothiazol-3-amine 1,1-dioxide, hydrochloride

A. 5-(Dimethylamino)methyl-2-furanmethanol

A mixture of 196 g (2 mol) of furfuryl alcohol, 245 g (3 mol) of dimethylamine hydrochloride and 200 mL of 37% formaldehyde is stirred at 0°C for 3 hours. The mixture is allowed to warm to room temperature and is stirred for 20 hours.

The mixture is treated with 750 g of sodium carbonate and the resulting slurry is extracted sequentially with ethyl acetate and acetone. The combined organic extracts are rotoevaporated and distilled to give in two fractions 209 g (67.3%) of the title compound.

*Fraction A* — b.p. 91—96°C [0.4 mm] 147.1 g
*Analysis for:* $C_8H_{13}NO_2$
*Calculated:* C, 61.91; H, 8.44; N, 9.03
*Found:* C, 61.25; H, 8.59; N, 8.86.

*Fraction B* — b.p. 96—98°C [0.4 mm] 61.9 g
*Analysis for:* $C_8H_{13}NO_2$
*Calculated:* C, 61.91; H, 8.44; N, 9.03
*Found:* C, 61.79; H, 8.41; N, 9.19.

B. 2-[[[5-(Dimethylamino)methyl-2-furanyl]methyl]thio]ethanamine

To a solution of 89.5 g (0.79 mol) of aminoethanethiol hydrochloride in 300 mL concentrated hydrochloric acid, maintained between −10 and 0°C, is added dropwise over 75 minutes, 116.4 g (0.75 mol) of 5-(dimethylamino)methyl-2-furanmethanol.

The mixture is placed under refrigeration (5°C) for 63 hours and then treated portionwise with 825 g of anhydrous sodium carbonate. The resulting slurry is exhaustively extracted with ethyl acetate and the organic extract is dried over magnesium sulfate and rotoevaporated. Distillation of the crude product gives in two fractions a total of 62.7 g (26.6%) of the title compound.

*Fraction A* — b.p. 122°C [0.5 mm] 34.4 g
*Analysis for:* $C_{10}H_{18}N_2OS$
*Calculated:* C, 56.04; H, 8.46; N, 13.07
*Found:* C, 55.66; H, 8.42; N, 12.92.

*Fraction B* — b.p. 123°C [0.5 mm] 28.3 g
*Analysis for:* $C_{10}H_{18}N_2OS$
*Calculated:* C, 56.04; H, 8.46; N, 13.07
*Found:* C, 55.76; H, 8.29; N, 12.99.

C. N-[2-[[[5-[(dimethylamino)methyl]-2-furanyl]methyl]thioethyl]thieno [3,4-d]isothiazol-3-amine 1,1-dioxide

To a suspension of 61.92 g (.28 mol) of 3-(methylthio)-thieno[3,4-*d*]isothiazol 1,1-dioxide in 800 mL of

absolute ethanol is added 60.52 g (.28 mol) of 2-[[[5-[(dimethylamino)methyl]-2-furanyl]methyl]thio]ethylamine. This mixture is stirred vigorously and is heated at reflux temperature for 2 hours. It forms a clear solution in about 10 minutes. When the heating is completed, the reaction is evaporated to dryness on a rotary evaporator, leaving a white solid residue. To this is added about 600 mL of ether and the residue is broken up until a finely divided solid results. This crude free base is collected on a suction filter and rinsed with more ether. This material is air-dried and amounts to 107 g (m.p. 106—9°C; yield 96%).

D. N-[2[[[5-[(dimethylamino)methyl]-2-furanyl]methyl]thio]ethyl]thieno[3,4-d]isothiazol-3-amine 1,1-dioxide, hydrochloride

The crude free base (109 g., .28 mol) of C above is dissolved in 1.5 litres of warm absolute ethanol and is then filtered by suction, and to this filtrate is added sufficient ethanolic HCl to obtain pH 1 (as determined by pH paper). The resulting clear yellow solution is cooled in ice and quickly begins precipitating the HCl salt. This suspension is further cooled to 5°C and then the crude product is collected on a suction filter and rinsed with 500 mL of cold absolute ethanol. The crude HCl salt is air-dried to give 118 g of product (m.p. 187°C, yield 99%).

Purification is accomplished by dissolving the crude product in 15 litres of absolute ethanol. The solution is concentrated to about 13 litres and cooled in a salt-ice bath to 5°C and then filtered on a suction filter. The product is rinsed with petroleum ether and dried in a vacuum oven at 90°C, giving 93 g of purified HCl salt (m.p. 189°C, 76% overall yield).

*Analysis for:* $C_{15}H_{20}ClN_3O_3S_3$
*Calculated:* C, 42.69; H, 4.78; N, 9.96
*Found:* C, 42.69; H, 4.82; N, 9.77.
HPLC purity 99.9%.

Example 8

N-[3-[4-(1-piperidinylmethyl)phenoxy]propyl]thieno[3,4-d]-isothiazol-3-amine 1,1-dioxide, methanesulfonate

A. 4-(1-Piperidinylmethyl)-phenol

To a solution of 60 g (0.48 mole) of 4-hydroxybenzaldehyde in 1000 ml of absolute ethanol is added 104 g (1.2 mole) of piperidine. The mixture is stirred at room temperature overnight and is then evaporated in a rotary evaporator. The residue is treated with 1000 ml of cold water and this mixture is acidified with concentrated hydrochloric acid to pH 1. The mixture is then extracted with 500 ml of ethyl acetate. The aqueous layer is basified with concentrated ammonium hydroxide. The precipitate which forms is collected and is air dried. This material is combined with the products from two other runs and this combination is recrystallised from ethanol to afford the first crop. The ethanol filtrate is evaporated and this residue is recrystallised from ethyl acetate to give a second crop. The two crops are combined to afford 148 g of product, m.p. 133—7°C. Yield 54%.

B. N-[3-[4-(1-piperidinylmethyl)phenoxy]propyl]-1H-isoindole-1,3(2H)-dione

To 250 ml of dry N,N-dimethylformamide is added 30.8 g (0.77 mole) of 60% sodium hydride. To this mixture is added a solution of 148 g (0.77 mole) of 4-(1-piperidinylmethyl)phenol in 300 ml of dry N,N-dimethylformamide dropwise over 45 minutes. After the addition is completed, the mixture is stirred at room temperature for 30 minutes. Then 210.7 g (0.77 mole) of 98% N-(3-bromopropyl)phthalimide is added. After stirring at room temperature for 3 hours, the mixture is poured into 3 litres of water. The mixture is extracted with 2000 ml of chloroform. The chloroform layer is washed with water and is dried over magnesium sulfate. After filtering, the filtrate is evaporated in a rotary evaporator. The residue is treated with 200 ml of water and this mixture is extracted with 500 ml of ether. The ether layer is dried over magnesium sulfate, filtered and evaporated. The residue is dissolved in 400 ml of anhydrous ether and the solution is cooled in ice. The precipitate which forms is collected to give 158 g of product, m.p. 72—5°C. Yield 54%.

A small amount of the material is recrystallised from heptane to give the analytical sample, m.p. 78—80°C.

*Analysis for:* $C_{23}H_{26}N_2O_3$
Calculated: C, 72.99; H, 6.93; N, 7.40
Found: C, 73.02; H, 6.89; N, 7.39.

C. 3-[4-(1-piperidinylmethyl)phenoxy]-1-propanamine

To a solution of 79 g (.21 mole) of *N*-[3-[4-(1-piperidinylmethyl)phenoxy]propyl-1*H*-isoindole-1,3(2*H*)-dione in 1600 ml of absolute ethanol is added 130 ml of hydrazine hydrate. The mixture is stirred at room temperature for 2 hours and the thick mixture is filtered. The filtrate is then stirred at room temperature for an additional two hours and is again filtered. This process is repeated a third time and the filtrate is finally left at room temperature overnight. The mixture is then filtered and the filtrate is evaporated in a rotary evaporator. The residue is triturated with 200 ml of chloroform and is filtered. The filtrate is dried over

magnesium sulfate and is evaporated after filtering. The residue is combined with a residue from a previous run and this combination is distilled to give 80 g of product, b.p. 148—52°C/0.35 mm. Yield 77%.

D. N-[3-[4-(1-piperidinylmethyl)phenoxy]propyl]-thieno[3,4-d]isothiazol-3-amine 1,1-dioxide

A stirred solution of 7.45 g (0.03 mole) of 3-[4-(1-piperidinylmethyl)-phenoxy]-1-propanamine and 5.62 g (0.03 mole) of 3-(methylthio)thieno[3,4-*d*]isothiazole 1,1-dioxide in 100 ml of ethanol is heated under reflux for 5 hours. The solution is cooled in ice. The resulting crystalline product is collected on a filter and amounts to 11.1 g (88.2%) m.p. 137—139°C, HPLC 98.5% pure.

E. N-[3-[4-(1-piperidinylmethyl)phenoxy]propyl]thieno[3,4-d]isothiazol-3-amine 1,1-dioxide methanesulfonate

To 2.061 g (0.0214 mole) of methanesulfonic acid in 125 ml of acetone is added 9.0 g (0.0214 mole) of *N*-[3-[4-(1-piperidinylmethyl)phenoxy]propyl]thieno[3,4-*d*]isothazole-3-amine 1,1-dioxide free base. After stirring for a few minutes a crystalline product is precipitated which amounts to 11.01 g, m.p. 212—213°C. Recrystallisation from ethanol gives 9.8 g (88.8%) of product, m.p. 212—214°C. HPLC 99.8% pure.

*Analysis for:* $C_{21}H_{29}N_3O_6S_3$
Calculated: C, 48.91; H, 5.67; N, 8.15
Found: C, 49.13; H, 5.76; N, 8.09.

**Claims for the Contracting States: BE CH DE FR GB IT LI LU NL SE**

1. A compound of general formula

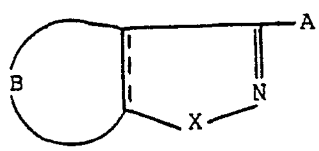

(I)

wherein B is a moiety having the formula:

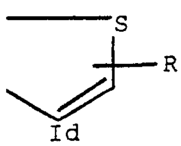

Id

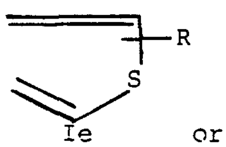

Ie

or

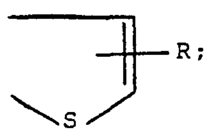

If

the dotted line represents a bond which is present when B is formula Id or If and absent when B is formula Ie, R is hydrogen, mono- or dihalo, amino, nitro, cyano, hydroxy, trifluoromethyl, mercapto, lower alkyl, lower alkoxy, alkanoyl of 2 to 5 carbon atoms, cycloalkyl of 5 to 7 carbon atoms, carboxy, alkoxycarbonyl, mono- or di-lower alkyl substituted amino, alkanoylamino of 2 to 5 carbon atoms, lower alkyl thio, loweralkylsulfonyl, sulfamoyl, lower alkyl substituted sulfamoyl, phenyl or phenyl substituted with halo, lower alkyl, lower alkoxy, trifluoromethyl, hydroxy, amino, cyano or nitro; X is $SO_2$, SO, S or C=O and A is an amine of formula:—

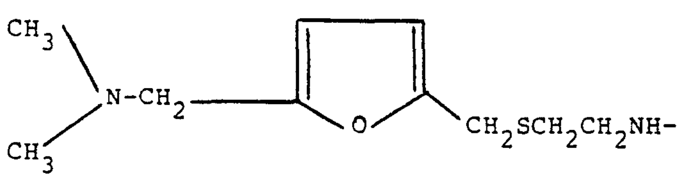

(Ia),

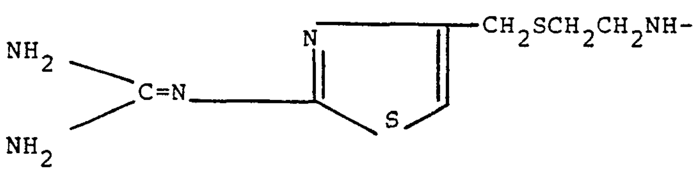

(Ib)

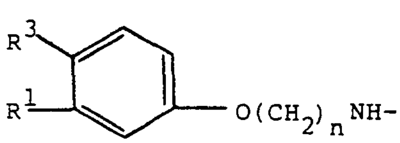

(Ic)

wherein n is 1 to 4; $R^1$ is hydrogen or $R^2CH_2$ wherein $R^2$ is mono- or diloweralkylamino, mono- or di-N-loweralkylamino lower alkyl, (2-furyl)methylamino, benzylamino, cycloalkylamino of 5 to 7 carbon atoms, 1-pyrrolidinyl, 1-piperidinyl, 1-hexahydroazepinyl, 1-octahydroazocinyl, 3-thiazolidinyl, 4-morpholinyl or 4-thiomorpholinyl; $R^3$ is hydrogen or (1-piperidinyl)-methyl with the proviso that when $R^3$ is (1-piperidinyl)-methyl, then $R^1$ is hydrogen; the term 'lower' as used herein to qualify 'alkyl' or 'alkoxy' means such groups contain 1 to 6 carbon atoms; and the pharmacologically acceptable salts thereof.

2. A compound as claimed in Claim 1 wherein A represents formula Ic or Ia.

3. A compound as claimed in Claim 1 or Claim 2 wherein B represents formula Ie.

4. A compound as claimed in any one of Claims 1 to 3 wherein $R^3$ is hydrogen.

5. A compound as claimed in any one of Claims 1 to 4 wherein n is 3.

6. A compound as claimed in any one of Claims 1 to 5 wherein $R^1$ is $R^2CH_2$ where $R^2$ is 1-piperidinyl.

7. A compound as claimed in any one of Claims 1 to 6 wherein X is $SO_2$.

8. A compound of formula I which is

N-[2-[[[5-[(Dimethylamino)methyl]-2-furanyl]methyl]-thio]ethyl]thieno[3,4-d]isothiazol-3-amine-1,1-dioxide; or

[4-[[[2-[(Thieno[3,4-*d*]isothiazol-3-yl]amino]ethyl]-thio]methyl]-2-thiazolyl]guanidine-S',S',-dioxide; or

*N*-[2-[3-(1-Piperidinylmethyl)phenoxy]propyl]thieno[3,4-d]isothiazol-3-amine-1,1-dioxide; or

*N*-[3-[4-(1-Piperidinylmethyl)phenoxy]propyl]thieno[3,4-*d*]isothiazol-3-amine-1,1-dioxide; or a pharmacologically acceptable salt thereof.

9. A compound as claimed in any one of Claims 1 to 8 when in the form of a salt with an acid selected from hydrochloric, hydrobromic, sulfuric, nitric, phosphoric, acetic, maleic, fumaric, citric and methanesulfonic.

10. A process for preparing a compound of formula I as defined in Claim 1 which comprises:

a) reacting a compound of formula

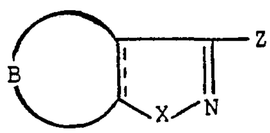

(II)

wherein the dotted line, X and B are as defined in Claim 1 and Z is $SR^4$ where $R^4$ is alkyl or aralkyl, with an amine of formula H—A wherein A is as defined in Claim 1 to give a compound of formula I, or

b) reacting a compound of formula

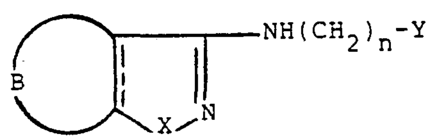

(III)

wherein the dotted line, X, B and n are as defined in Claim 1 and Y is OH or L where L represents a leaving group or an equivalent replaceable group, with a compound of formula:

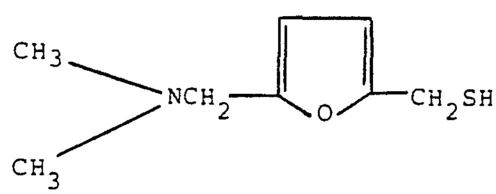

(IVa)

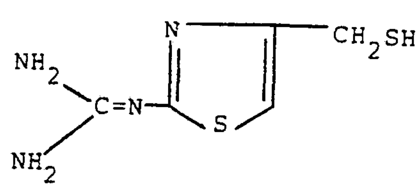

(IVb)

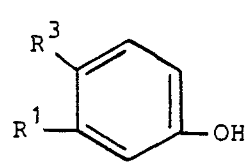

(IVc)

wherein $R^1$ and $R^3$ are as defined in Claim 1, with the proviso that (i) when a compound of formula IVa or IVb is used then n is 2 in the compound of formula III and (ii) when a compound of formula IVc is used then Y is L; or

c) reacting a compound of formula

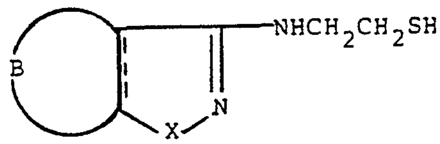

(V)

wherein the dotted line, B and X are as defined in Claim 1, with a compound of formula:

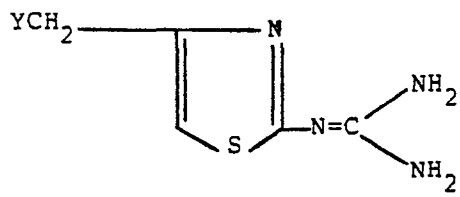

(VIa)

or

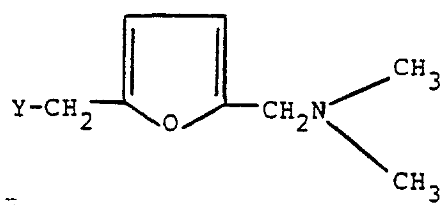

(VIb)

wherein Y is OH or L wherein L is a leaving group to give a compound of formula I wherein A has the formula Ia or Ib as defined in Claim 1; or

d) reacting an aldehyde of formula

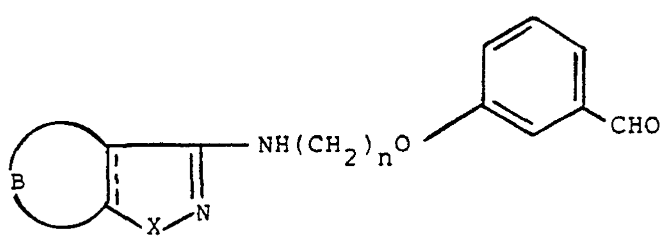

(VIIa)

with an appropriate amine of formula

$$HR^2 \qquad (VIIb)$$

in which formulae the dotted line, n, B, X and $R^2$ are as defined in Claim 1, under reductive amination conditions to give a corresponding compound of formula I wherein $R^1$ is $R^2CH_2$— and $R^3$ is hydrogen; or

e) reacting an aldehyde of formula

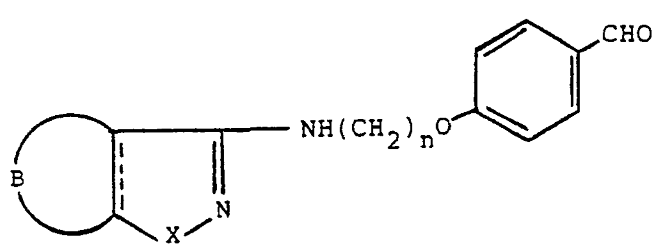

(VIIc)

wherein the dotted line, n, B and X are as defined in Claim 1 with piperidine under reductive amination conditions to give a compound of formula I wherein $R^3$ is (1-piperidinyl)methyl; or

f) reacting an aldehyde of formula

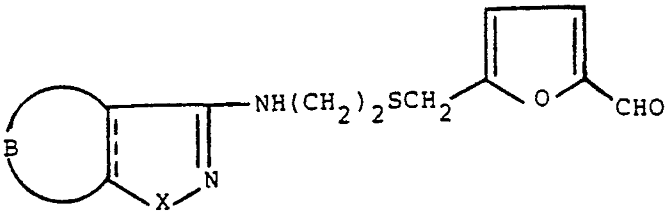

(VIId)

wherein the dotted line, B and X are as defined in Claim 1 with dimethylamine under reductive amination conditions to give a compound of formula I wherein A has formula Ia as defined in Claim 1; or

g) reacting a compound of formula

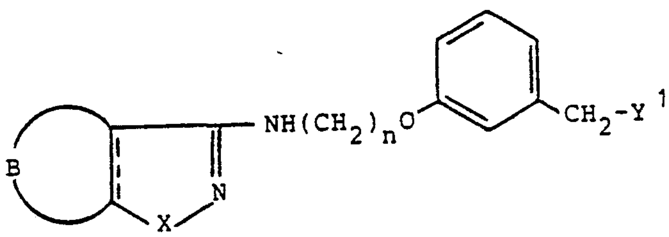

(VIII)

wherein the dotted line, n, X and B are as defined in Claim 1 and $Y^1$ is OH or L wherein L is a leaving group, with an amine of formula (VIIb)

$$HR^2$$

wherein $R^2$ as defined in Claim 1, the reaction being carried out in the presence of a nickel catalyst when $Y^1$ is OH, to give a compound of formula I wherein A has the formula Ic and $R^1$ is $R^2CH_2$—; or

h) reacting a compound of formula

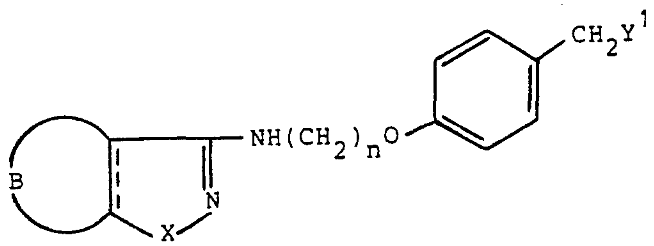

(VIIIa)

wherein the dotted line, $Y^1$, n, X and B are as defined in Claim 1, with piperidine, the reaction being carried out in the presence of a nickel catalyst when $Y^1$ is OH, to give a compound of formula I wherein A has the formula Ic as defined in Claim 1 and $R^3$ is (1-piperidinyl)methyl; or

i) reacting a compound of formula

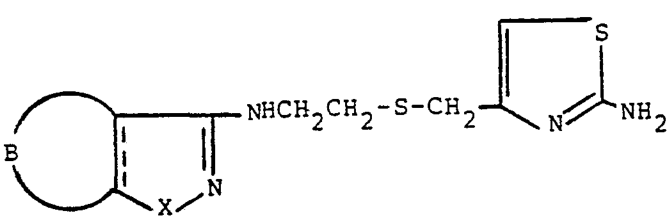

(IX)

wherein the dotted line, X and B are as defined in Claim 1 with a compound capable of converting —$NH_2$ to

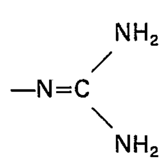

to give a compound of formula I wherein A has formula Ic, as defined in Claim 1, or

j) reacting a compound of formula

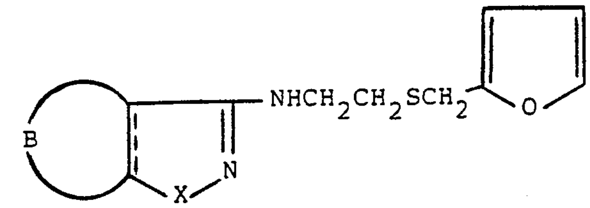

(X)

wherein the dotted line, X and B are as defined in Claim 1 with dimethylamine in the presence of formaldehyde or paraformaldehyde, to give a compound of formula I wherein A has formula Ia as defined in Claim 1, or

k) reacting a compound of formula

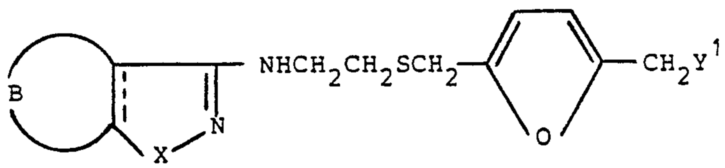

(XI)

wherein $Y^1$ is OH or a leaving group and the dotted line, X and B are as defined in Claim 1, with dimethylamine; the reaction being carried out in the presence of a nickel catalyst when $Y^1$ is OH; or

l) a compound of formula I as defined in Claim 1 having a reactive substituent group is converted to a compound of formula I having a different substituent group; or

m) a basic compound of formula I as defined in Claim 1 is converted to a pharmacologically acceptable salt or a pharmacologically acceptable salt is converted to the free base form.

11. A compound of formula I or a pharmacologically acceptable salt thereof as defined in any one of Claims 1 to 9 for use as a pharmaceutical.

12. A pharmaceutical composition comprising a compound of formula I or a pharmacologically acceptable salt as defined in any one of Claims 1—9 and a pharmaceutically acceptable carrier.

**Claims for the Contracting State: AT**

1. A process for preparing a compound of general formula

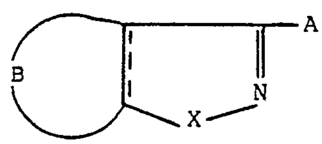

(I)

wherein B is a moiety having the formula:

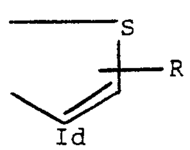

Id

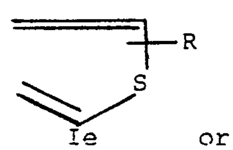

Ie

or

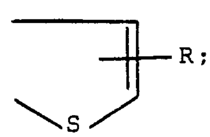

If

the dotted line represents a bond which is present when B is formula Id or If and absent when B is formula Ie, R is hydrogen, mono- or dihalo, amino, nitro, cyano, hydroxy, trifluoromethyl, mercapto, lower alkyl, lower alkoxy, alkanoyl of 2 to 5 carbon atoms, cycloalkyl of 5 to 7 carbon atoms, carboxy, alkoxycarbonyl, mono- or di-lower alkyl substituted amino, alkanoylamino of 2 to 5 carbon atoms, lower alkyl thio, loweralkylsulfonyl, sulfamoyl, lower alkyl substituted sulfamoyl, phenyl or phenyl substituted with halo, lower alkyl, lower alkoxy, trifluoromethyl, hydroxy, amino, cyano or nitro; X is $SO_2$, SO, S or C=O and A is an amine of formula:—

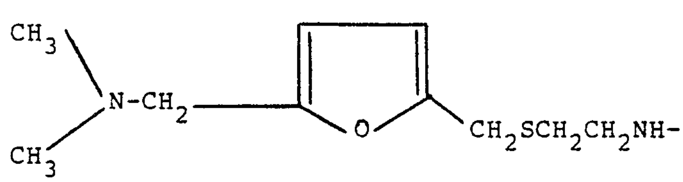

(Ia),

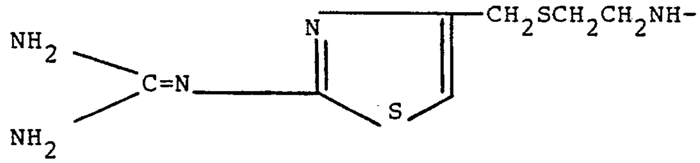

(Ib)

or

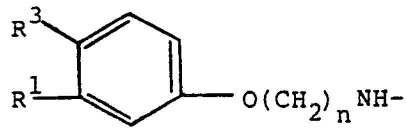

(Ic)

wherein n is 1 to 4; $R^1$ is hydrogen or $R^2CH_2$ wherein $R^2$ is mono- or diloweralkylamino, mono- or di-N-loweralkylamino lower alkyl, (2-furyl)methylamino, benzylamino, cycloalkylamino of 5 to 7 carbon atoms, 1-pyrrolidinyl, 1-piperidinyl, 1-hexahydroazepinyl, 1-octahydroazocinyl, 3-thiazolidinyl, 4-morpholinyl or 4-thiomorpholinyl; $R^3$ is hydrogen or (1-piperidinyl)-methyl with the proviso that when $R^3$ is (1-piperidinyl)-methyl, then $R^1$ is hydrogen; the term 'lower' as used herein to qualify 'alkyl' or 'alkoxy' means such groups contain 1 to 6 carbon atoms; and the pharmacologically acceptable salts thereof, which comprises

a) reacting a compound of formula

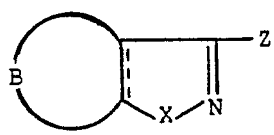

(II)

wherein the dotted line, X and B are as defined above and Z is $SR^4$ where $R^4$ is alkyl or aralkyl, with an amine of formula H—A wherein A is as defined above to give a compound of formula I, or

b) reacting a compound of formula

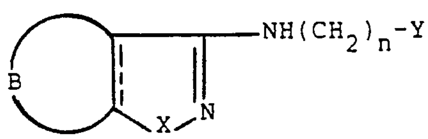

(III)

wherein the dotted line, X, B and n are as defined above and Y is OH or L where L represents a leaving group or an equivalent replaceable group, with a compound of formula:

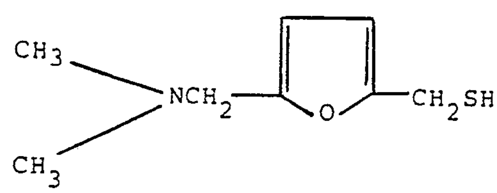

(IVa)

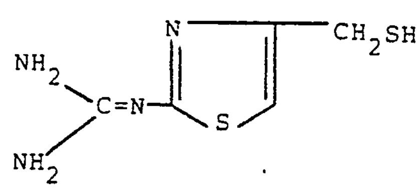

(IVb)

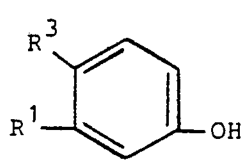

(IVc)

wherein $R^1$ and $R^3$ are as defined above, with the proviso that (i) when a compound of formula IVa or IVb is used then n is 2 in the compound of formula III and (ii) when a compound of formula IVc is used then Y is L; or

c) reacting a compound of formula

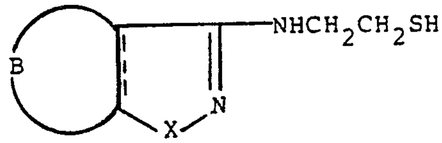

(V)

wherein the dotted line, B and X are as defined above, with a compound of formula:

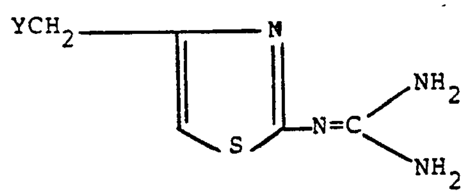

(VIa)

or

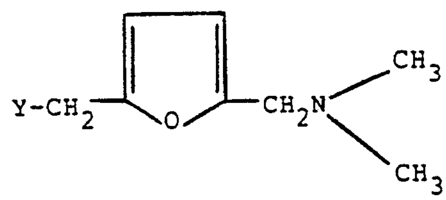

(VIb)

wherein Y is OH or L wherein L is a leaving group to give a compound of formula I wherein A has the formula Ia or Ib as defined above; or

d) reacting an aldehyde of formula

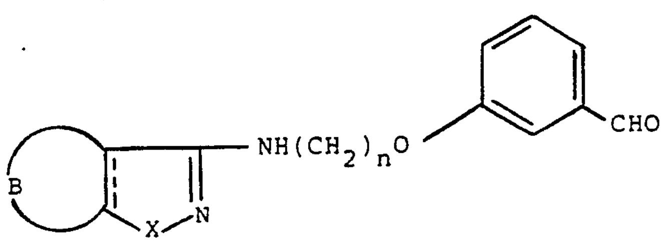

(VIIa)

with an appropriate amine of formula

$HR^2$ (VIIb)

in which formulae the dotted line, n, B, X and $R^2$ are as defined above, under reductive amination conditions to give a corresponding compound of formula I wherein $R^1$ is $R^2CH_2$— and $R^3$ is hydrogen; or

e) reacting an aldehyde of formula

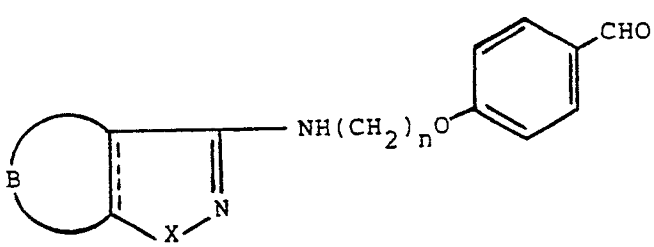

(VIIc)

wherein the dotted line, n, B and X are as defined above with piperidine under reductive amination conditions to give a compound of formula I wherein $R^3$ is (1-piperidinyl)methyl; or

f) reacting an aldehyde of formula

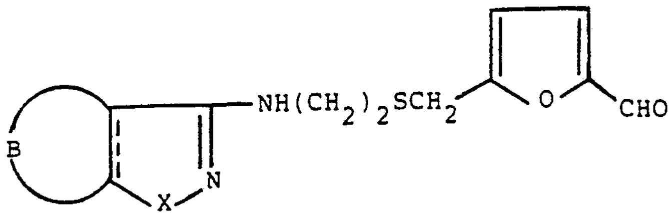

(VIId)

wherein the dotted line, B and X are as defined above with dimethylamine under reductive amination conditions to give a compound of formula I wherein A has formula Ia as defined above; or

g) reacting a compound of formula

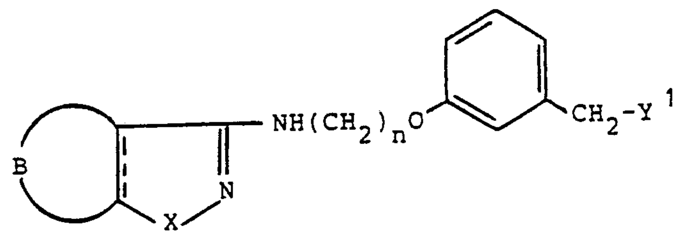

(VIII)

wherein the dotted line, n, X and B are as defined above and $Y^1$ is OH or L wherein L is a leaving group, with an amine of formula (VIIb)

$$HR^2$$

wherein $R^2$ as defined above, the reaction being carried out in the presence of a nickel catalyst when $Y^1$ i OH, to give a compound of formula I wherein A has the formula Ic and $R^1$ is $R^2CH_2$—; or

h) reacting a compound of formula

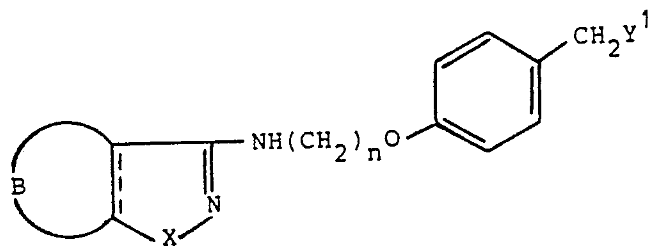

(VIIIa)

wherein the dotted line, $Y^1$, n, X and B are as defined above, with piperidine, the reaction being carried out in the presence of a nickel catalyst when $Y^1$ is OH, to give a compound of formula I wherein A has the formula Ic as defined above and $R^3$ is (1-piperidinyl)methyl; or

i) reacting a compound of formula

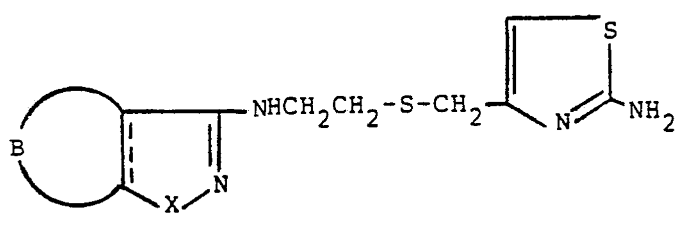

(IX)

wherein the dotted line, X and B are as defined above with a compound capable of converting $—NH_2$ to

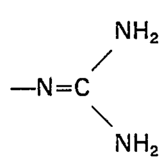

to give a compound of formula I wherein A has formula Ic, as defined above, or

j) reacting a compound of formula

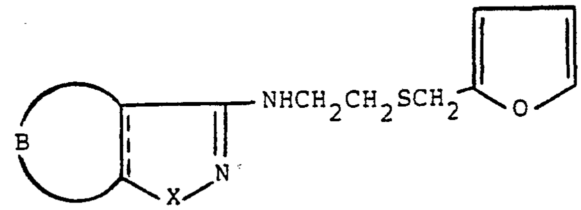

(X)

wherein the dotted line, X and B are as defined above with dimethylamine in the presence of formaldehyde or paraformaldehyde, to give a compound of formula I wherein A has formula Ia as defined above, or

k) reacting a compound of formula

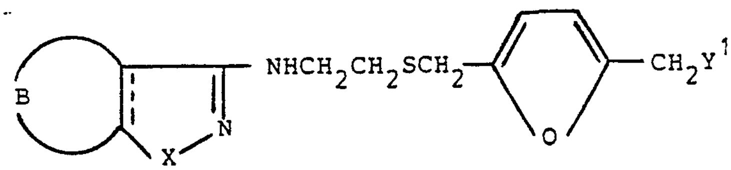

(XI)

wherein $Y^1$ is OH or a leaving group and the dotted line, X and B are as defined above, with dimethylamine; the reaction being carried out in the presence of a nickel catalyst when $Y^1$ is OH; or

l) a compound of formula I as defined above having a reactive substituent group is converted to a compound of formula I having a different substituent group; or

m) a basic compound of formula I as defined above is converted to a pharmacologically acceptable salt or a pharmacologically acceptable salt is converted to the free base form.

2. A process as claimed in Claim 1 wherein A represents formula Ic or Ia.

3. A process as claimed in Claim 1 or Claim 2 wherein B represents formula Ie.

4. A process as claimed in any one of Claims 1 to 3 wherein $R^3$ is hydrogen.

5. A process as claimed in any one of Claims 1 to 4 wherein n is 3.

6. A process as claimed in any one of Claims 1 to 5 wherein $R^1$ is $R^2CH_2$ where $R^2$ is 1-piperidinyl.

7. A process as claimed in any one of Claims 1 to 6 wherein X is $SO_2$.

8. A process as claimed in Claim 1 in which the compound prepared is

N-[2-[[[5-[(Dimethylamino)methyl]-2-furanyl]methyl]-thio]ethyl]thieno[3,4-d]isothiazol-3-amine-1,1-dioxide, or

[4-[[[2-[(Thieno[3,4-*d*]isothiazol-3-yl]amino]ethyl]-thio]methyl]-2-thiazolyl]guanidine-S',S',-dioxide, or

*N*-[2-[3-(1-Piperidinylmethyl)phenoxy]propyl]thieno[3,4-d]isothiazol-3-amine-1,1-dioxide, or

*N*-[3-[4-(1-Piperidinylmethyl)phenoxy]propyl]thieno[3,4-*d*]isothiazol-3-amine-1,1-dioxide; or a pharmacologically acceptable salt thereof.

9. A process for preparing a pharmaceutical composition characterised in that a compound of formula I as defined in any one of Claims 1 to 8 or a pharmacologically acceptable salt thereof is mixed with a pharmaceutical carrier and brought into a form suitable for therapeutic administration.

10. A process as claimed in Claim 9 in which the compound of formula I is prepared by a process as claimed in any one of Claims 1 to 8.

**Patentansprüche für die Vertragsstaaten: BE CH DE FR GB IT LI LU NL und SE**

1. Verbindung der allgemeinen Formel

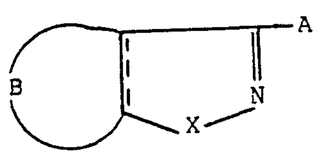

(I)

worin B eine Gruppe der Formel

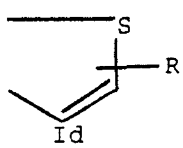

Id

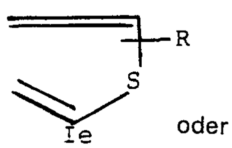

Ie

oder

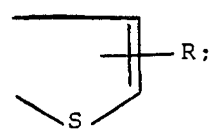

If

ist; die strichlierte Linie eine Bindung darstellt, die vorhanden ist, wenn B Formel (Id) oder (If) aufweist, und

fehlt, wenn B die Formel (Ie) aufweist, R Wasserstoff, Mono- oder Dihalogen, Amino, Nitro, Cyano, Hydroxy, Trifluormethyl, Mercapto, nied.Alkyl, nied.Alkoxy, Alkanoyl mit 2 bis 5 C-Atomen, Cycloalkyl mit 5 bis 7 C-Atomen, Alkoxycarbonyl, mono- oder di-nied.alkylsubstituiertes Amino, Alkanoylamino mit 2 bis 5 C-Atomen, nied.Alkylthio, nied.Alkylsulfonyl, Sulfamoyl, nied.alkylsubstituiertes Sulfamoyl, Phenyl oder Phenyl substituert mit Halogen, nied.Alkyl, nied.Alkoxy, Trifluormethyl, Hydroxy, Amino, Cyano oder Nitro bedeutet; X $SO_2$, SO, S oder C=O darstellt und A ein Amin der Formel

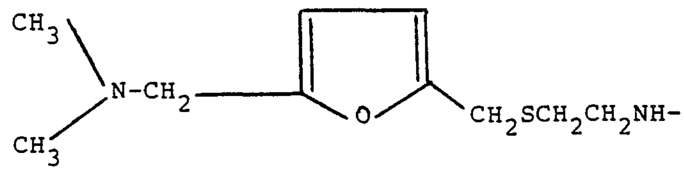

(Ia),

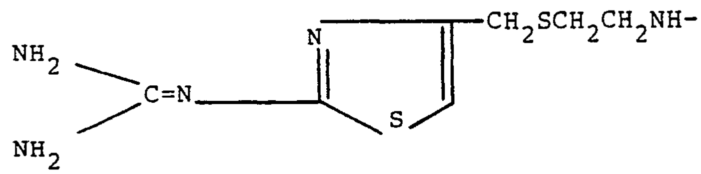

(Ib)

or

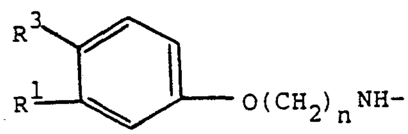

(Ic)

ist, worin n 1 bis 4 ist; R[1] Wasserstoff oder $R^2CH_2$ bedeutet, wobei $R_2$ Mono- oder Di-nied.alkylamino, Mono- oder Di-N-nied.alkylamino-nied.alkyl, (2-Furyl)-methylamino, Benzylamino, Cycloalkylamino mit 5 bis 7 C-Atomen, 1-Pyrrolidinyl, 1-Piperidinyl, 1-Hexahydroazepinyl, 1-Octahydroazocinyl, 3-Thiazolidinyl, 4-Morpholinyl oder 5-Thiomorpholinyl ist; $R^3$ Wasserstoff oder (1-Piperidinyl)-methyl bedeutet, mit der Maßgabe, daß, wenn $R^3$ (1-Piperidinyl)-methyl ist, $R^1$ Wasserstoff darstellt, wobei der hier verwendete Ausdruck "Alkyl" oder "Alkoxy" bedeutet, daß derartige Gruppen 1 bis 6 C-Atome enthalten, und deren pharmakologisch annehmbare Salze.

2. Verbindung, wie in Anspruch 1 beansprucht, worin A die Formel (Ic) oder (Ia) darstellt.

3. Verbindung, wie in Anspruch 1 oder Anspruch 2 beansprucht, worin B die Formel (Ie) darstellt.

4. Verbindung, wie in einem der Ansprüche 1 bis 3 beansprucht, worin $R^3$ Wasserstoff ist.

5. Verbindung, wie in einem der Ansprüche 1 bis 4 beansprucht, worin n 3 ist.

6. Verbindung, wie in einem der Ansprüche 1 bis 5 beansprucht, worin $R^1$ $R^2CH_2$ bedeutet, wobei $R^2$ 1-Piperidinyl ist.

7. Verbindung, wie in einem der Ansprüche 1 bis 6 beansprucht, worin X $SO_2$ ist.

8. Verbindung der Formel (I), die

N-[2-[[[5-[(Dimethylamino)-methyl]-2-furanyl]-methyl]-thio]-äthyl]-thieno[3,4-d]isothiazol-3-amin-1,1-dioxid oder

[4-[[[2-[(Thieno[3,4-d]isothiazol-3-yl)-amino]-äthyl]-thio]-methyl]-2-thiazolyl]-guanidin-S',S'-dioxid oder

N-[2-[3-(1-Piperidinylmethyl)-phenoxy]-propyl]-thieno[3,4-d]isothiazol-3-amin-1,1-dioxid oder

N-[3-[4-(1-Piperidinylmethyl)-phenoxy]-propyl]-thieno[3,4-d]isothiazol-3-amin-1,1-dioxid oder

ein pharmakologisch annehmbares Salz hievon ist.

9. Verbindung, wie in einem der Ansprüche 1 bis 8 beansprucht, wenn sie in Form eines Salzes mit einer Säure ausgewählt aus Salz-, Bromwasserstoff-, Schwefel-, Salpeter-, Phosphor-, Essig-, Malein-, Fumar-, Citronen- und Methansulfonsäure ist.

10. Verfahren zum Herstellen einer Verbindung der Formel (I), wie in Anspruch 1 definiert, welches umfaßt:

a) das Umsetzen einer Verbindung der Formel

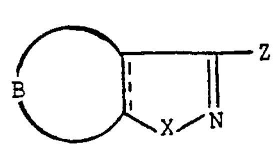

(II)

worin die strichlierte Linie, X und B wie in Anspruch 1 definiert sind und Z $SR^4$ ist, wobei $R^4$ Alkyl oder Aralkyl bedeutet, mit einem Amin der Formel H—A, worin A wie in Anspruch 1 definierte ist, wobei eine Verbindung der Formel (I) erhalten wird; oder

b) das Umsetzen einer Verbindung der Formel

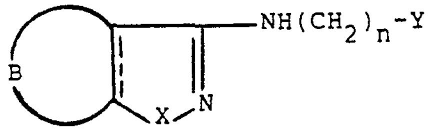

(III)

worin die strichlierte Linie, X, B und n wie in Anspruch 1 definiert sind und Y OH oder L darstellt, wobei L eine abspaltbare Gruppe oder eine äquivalente ersetzbare Gruppe ist, mit einer Verbindung der Formel

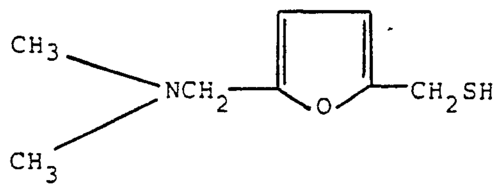

(IVa)

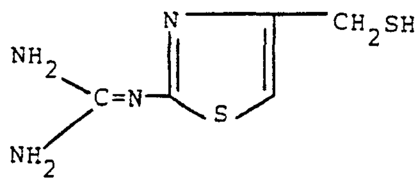

(IVb)

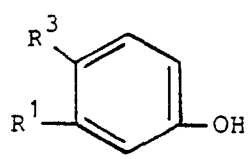

(IVc)

worin $R^1$ und $R^3$ wie in Anspruch 1 definiert sind, mit der Maßgabe, daß (i) wenn eine Verbindung der Formel (IVa) oder (IVb) verwendet wird, n in der Verbindung der Formel (III) 2 ist, und (ii) wenn eine Verbindung der Formel (IVc) verwendet wird, Y die Bedeutung L hat; oder

c) das Umsetzen einer Verbindung der Formel

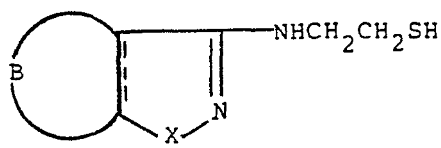

(V)

worin die strichlierte Linie, B und X wie in Anspruch 1 definiert sind, mit einer Verbindung der Formel

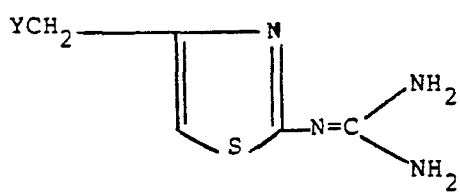

(VIa)

or

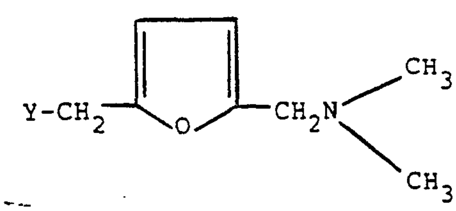

(VIb)

worin Y die Bedeutung OH oder L hat, wobei L eine abspaltbare Gruppe ist, wobei eine Verbindung der Formel (I) erhalten wird, worin A die Formel (Ia) oder (Ib), wie in Anspruch 1 definiert, aufweist; oder

d) das Umsetzen eines Aldehyds der Formel

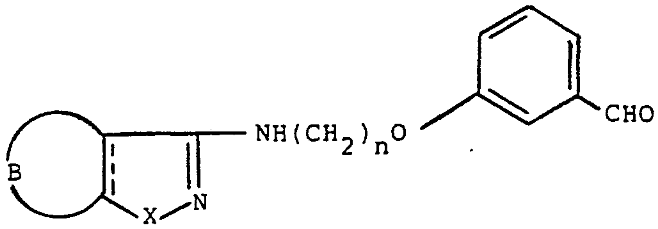

(VIIa)

mit einem geeigneten Amin der Formel

$HR^2$ (VIIb),

in welchen Formeln die strichlierte Linie, n, B, X und $R^2$ wie in Anspruch 1 definiert sind, unter reduktiven Aminiertungsbedingungen, wobei eine entsprechende Verbindung der Formel (I) erhalten wird, worin $R^1$ die Bedeutung $R^2CH_2$— hat und $R^3$ Wasserstoff ist; oder

e) das Umsetzen eines Aldehyds der Formel

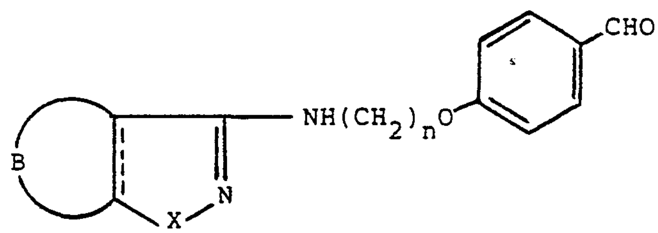

(VIIc)

worin die strichlierte Linie, n, B und X wie in Anspruch 1 definiert sind, mit Piperidin unter reduktiven Aminierungsbedingungen, wobei eine Verbindung der Formel (I) erhalten wird, worin $R^3$ (1-Piperidinyl)-methyl ist; oder

f) das Umsetzen eines Aldehyds der Formel

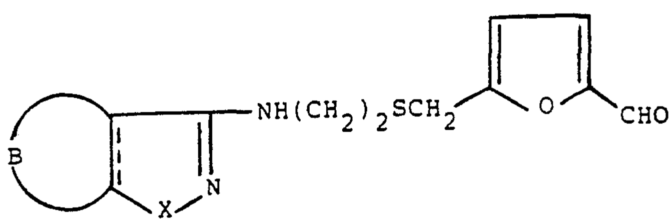

(VIId)

worin die strichlierte Linie, B und X wie in Anspruch 1 definiert sind, mit Dimethylamin unter reduktiven Aminierungsbedingungen, wobei eine Verbindung der Formel (I) erhalten wird, worin A die Formel (Ia), wie in Anspruch 1 definiert, aufweist; oder

g) das Umsetzen einer Verbindung der Formel

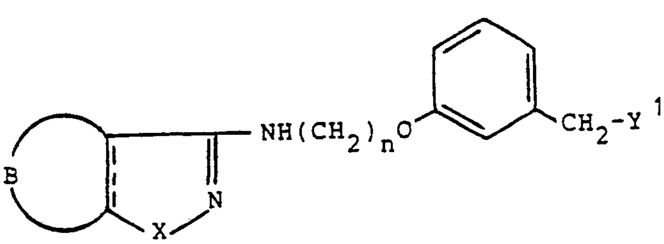

(VIII)

worin die strichlierte Linie, n, X und B wie in Anspruch 1 definiert sind und $Y^1$ OH oder L bedeutet, wobei L eine abspaltbare Gruppe ist, mit einem Amin der Formel $HR^2$ (VIIb), worin $R^2$ wie in Anspruch 1 definiert ist, wobei die Umsetzung in Anwesenheit eines Nickelkatalysators durchgeführt wird, wenn $Y^1$ OH ist, wobei eine Verbindung der Formel (I) erhalten wird, worin A die Formel (Ic) aufweist und $R^1$ $R^2CH_2$— ist; oder

h) das Umsetzen einer Verbindung der Formel

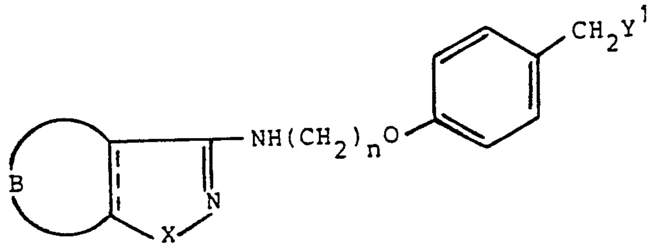

(VIIIa)

worin die strichlierte Linie, $Y^1$, n, X und B wie in Anspruch 1 definiert sind, mit Piperidin, wobei die Reaktion in Anwesenheit eines Nickelkatalysators durchgeführt wird, wenn $Y^1$ OH ist, wobei eine Verbindung der Formel (I) erhalten wird, worin A die Formel (Ic), wie in Anspruch 1 definiert, aufweist und $R^3$ (1-Piperidinyl)-methyl ist; oder

i) das Umsetzen einer Verbindung der Formel

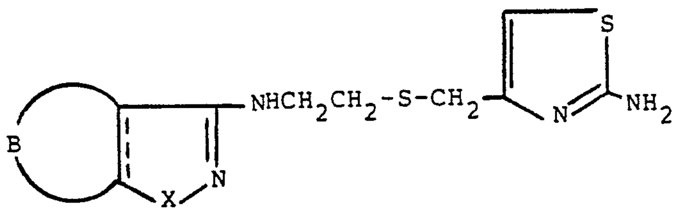

(IX)

worin die strichlierte Linie, X und B wie in Anspruch 1 definiert sind, mit einer Verbindung, die imstande ist,

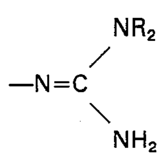

überzufuhren, wobei eine Verbindung der Formel (I) erhalten wird, worin A die Formel (Ic), wie in Anspruch 1 definiert, aufweist;

j) das Umsetzen einer Verbindung der Formel

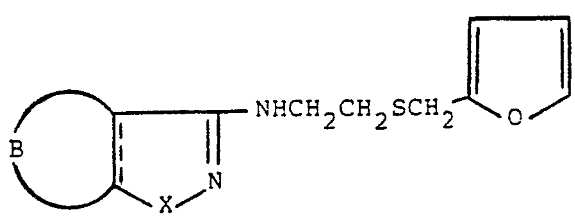

(X)

worin die strichlierte Linie, X und B wie in Anspruch 1 definiert sind, mit Dimethylamin in Anwesenheit von Formaldehyd oder p-Formaldehyd, wobei eine Verbindung der Formel (I) erhalten wird, worin A die Formel (Ia), wie in Anspruch 1 definiert, aufweist; oder

k) das Umsetzen einer Verbindung der Formel

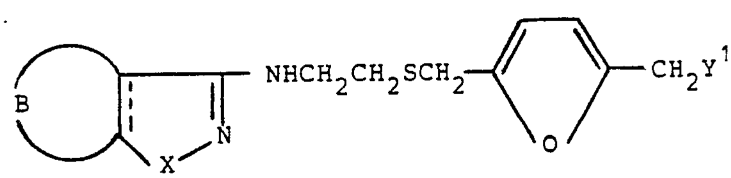

(XI)

worin $Y^1$ OH oder eine abspaltbare Gruppe darstellt und die strichlierte Linie, L und B wie in Anspruch 1 definiert sind, mit Dimethylamin, wobei die Reaktion in Anwesenheit eines Nickelkatalysators durchgeführt wird, wenn $Y^1$ OH ist; oder

l) die Überführung einer Verbindung der Formel (I), wie in Anspruch 1 definiert, mit einer reaktiven Substituentengruppe in eine Verbindung der Formel (I) mit einer anderen Substituentengruppe; oder

m) die Überführung einer basischen Verbindung der Formel (I), wie in Anspruch 1 definiert, in ein

pharmakologisch annehmbares Salz oder eines pharmakologisch annehmbaren Salzes in die Form der freien Base.

11. Verbindung der Formel (I) oder ein pharmakologisch annehmbares Salz hievon, wie in einem der Ansprüche 1 bis 9 definiert, zur Verwendung als Pharmazeutikum.

12. Pharmazeutische Zusammensetzung, die eine Verbindung der Formel (I) oder ein pharmakologisch annehmbares Salz, wie in einem der Ansprüche 1 bis 9 definiert, und einen pharmazeutisch annehmbaren Träger umfaßt.

**Patentansprüche für den Vertragsstaat: AT**

1. Verfahren zum Herstellen einer Verbindung der allgemeinen Formel

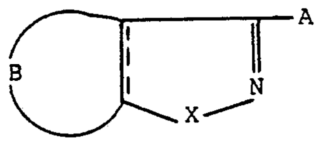

(I)

worin B eine Gruppe der Formel

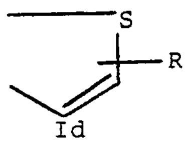

Id

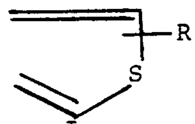

Ie

oder

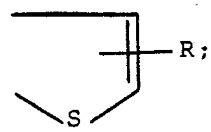

If

ist; die strichlierte Linie eine Bindung darstellt, die vorhanden ist, wenn B Formel (Id) oder (If) aufweist, und fehlt, wenn B die Formel (Ie) aufweist, R Wasserstoff, Mono- oder Dihalogen, Amino, Nitro, Cyano, Hydroxy, Trifluormethyl, Mercapto, nied.Alkyl, nied.Alkoxy, Alkanoyl mit 2 bis 5 C-Atomen, Cycloalkyl mit 5 bis 7 C-Atomen, Alkoxycarbonyl, mono- oder di-nied.alkylsubstituiertes Amino, Alkanoylamino mit 2 bis 5 C-Atomen, nied.Alkylthio, nied.Alkylsulfonyl, Sulfamoyl, nied.alkylsubstituiertes Sulfamoyl, Phenyl oder Phenyl substituiert mit Halogen, nied.Alkyl, nied.Alkoxy, Trifluormethyl, Hydroxy, Amino, Cyano oder Nitro bedeutet; X $SO_2$, SO, S oder C=O darstellt und A ein Amin der Formel

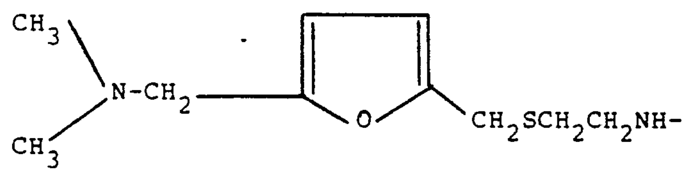

(Ia),

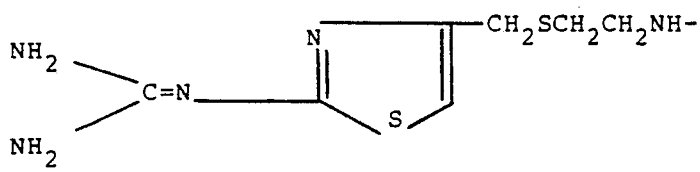

(Ib)

or

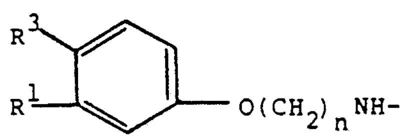

(Ic)

ist, worin n 1 bis 4 ist; $R^1$ Wasserstoff oder $R^2CH_2$ bedeutet, wobei $R_2$ Mono- oder Di-nied.alkylamino, Mono- oder Di-N-nied.alkylamino-nied.alkyl, (2-Furyl)-methylamino, Benzylamino, Cycloalkylamino mit 5 bis 7 C-Atomen, 1-Pyrrolidinyl, 1-Piperidinyl, 1-Hexahydroazepinyl, 1-Octahydroazocinyl, 3-Thiazolidinyl, 4-Morpholinyl oder 4-Thiomorpholinyl ist; $R^3$ Wasserstoff oder (1-Piperidinyl)-methyl bedeutet, mit der Maßgabe, daß, wenn $R^3$ (1-Piperidinyl)-methyl ist, $R^1$ Wasserstoff darstellt, wobei der hier verwendete Ausdruck "Alkyl" oder "Alkoxy" bedeutet, daß derartige Gruppen 1 bis 6 C-Atome enthalten, und der pharmakologisch annehmbare Salze hievon, welches umfaßt:

a) das Umsetzen einer Verbindung der Formel

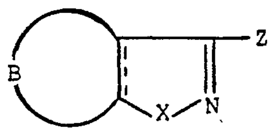

(II)

worin die strichlierte Linie, X und B wie oben definiert sind und Z SR[4] ist, wobei R[4] Alkyl oder Aralkyl bedeutet, mit einem Amin der Formel H—A, worin A wie oben definierte ist, wobei eine Verbindung der Formel (I) erhalten wird; oder

b) das Umsetzen einer Verbindung der Formel

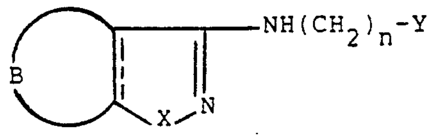

(III)

worin die strichlierte Linie, X, B und n wie oben definiert sind und Y OH oder L darstellt, wobei L eine abspaltbare Gruppe oder eine äquivalente ersetzbare Gruppe ist, mit einer Verbindung der Formel

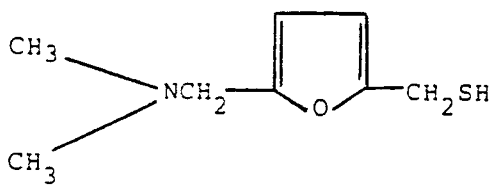

(IVa)

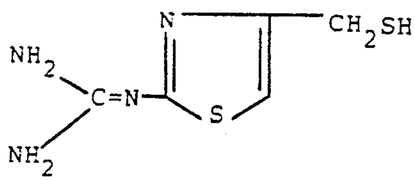

(IVb)

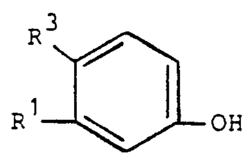

(IVc)

worin $R^1$ und $R^3$ wie oben definiert sind, mit der Maßgabe, daß (i) wenn eine Verbindung der Formel (IVa) oder (IVb) verwendet wird, n in der Verbindung der Formel (III) 2 ist, und (ii) wenn eine Verbindung der Formel (IVc) verwendet wird, Y die Bedeutung L hat; oder

c) das Umsetzen einer Verbindung der Formel

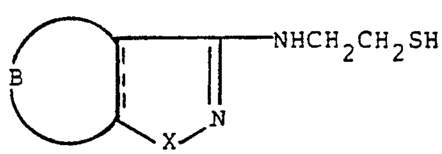

(V)

worin die strichlierte Linie, B und X wie oben definiert sind, mit einer Verbindung der Formel

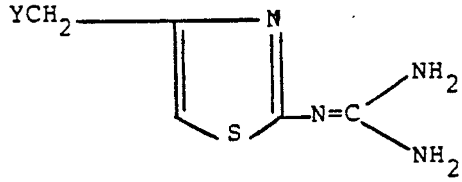

(VIa)

or

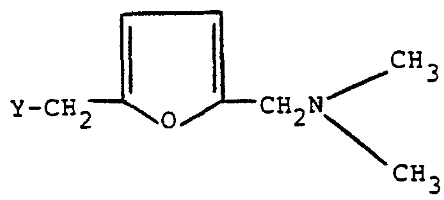

(VIb)

worin Y die Bedeutung OH oder L hat, wobei L eine abspaltbare Gruppe ist, wobei eine Verbindung der Formel (I) erhalten wird, worin A die Formel (Ia) oder (Ib), wie oben definiert, aufweist; oder

d) das Umsetzen eines Aldehyds der Formel

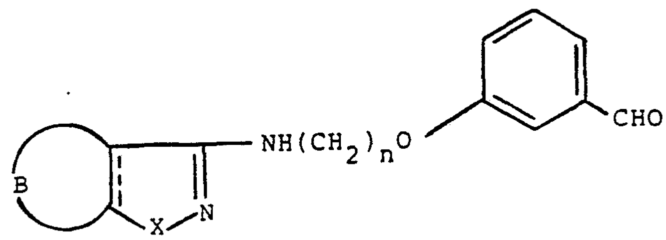

(VIIa)

mit einem geeigneten Amin der Formel

$HR^2$ (VIIb),

in welchen Formeln die strichlierte Linie, n, B, X und $R^2$ wie oben definiert sind, unter reduktiven Aminiertungsbedingungen, wobei eine entsprechende Verbindung der Formel (I) erhalten wird, worin $R^1$ die Bedeutung $R^2CH_2$— hat und $R^3$ Wasserstoff ist; oder

e) das Umsetzen eines Aldehyds der Formel

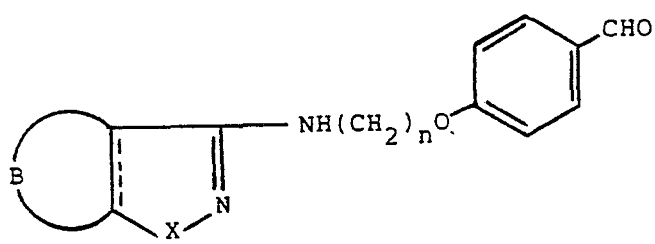

(VIIc)

worin die strichlierte Linie, n, B und X wie oben definiert sind, mit Piperidin unter reduktiven Aminierungsbedingungen, wobei eine Verbindung der Formel (I) erhalten wird, worin $R^3$ (1-Piperidinyl)-methyl ist; oder

f) das Umsetzen eines Aldehyds der Formel

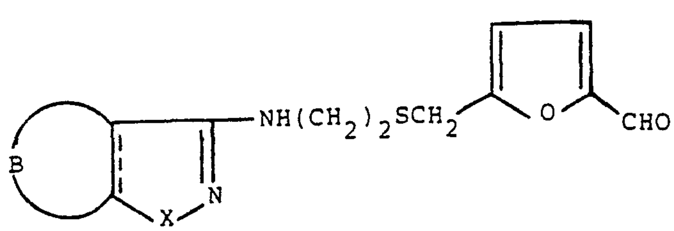

(VIId)

worin die strichlierte Linie, B und X wie oben definiert sind, mit Dimethylamin unter reduktiven Aminierungsbedingungen, wobei eine Verbindung der Formel (I) erhalten wird, worin A die Formel (Ia), wie oben definiert, aufweist; oder

g) das Umsetzen einer Verbindung der Formel

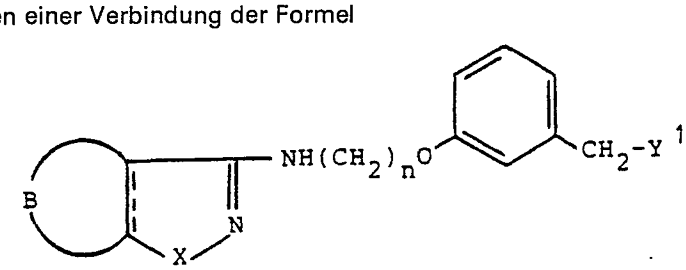

(VIII)

worin die strichlierte Linie, n, X und B wie oben definiert sind und $Y^1$ OH oder L bedeutet, wobei L eine abspaltbare Gruppe ist, mit einem Amin der Formel $HR^2$ (VIIb), worin $R^2$ wie oben definiert ist, wobei die Umsetzung in Anwesenheit eines Nickelkatalysators durchgeführt wird, wenn $Y^1$ OH ist, wobei eine Verbindung der Formel (I) erhalten wird, worin A die Formel (Ic) aufweist und $R^1$ $R^2CH_2$— ist; oder

h) das Umsetzen einer Verbindung der Formel

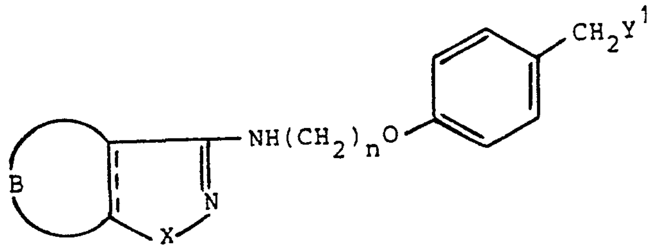

(VIIIa)

worin die strichlierte Linie, $Y^1$, n, X und B wie oben definiert sind, mit Piperidin, wobei die Reaktion in Anwesenheit eines Nickelkatalysators durchgeführt wird, wenn $Y^1$ OH ist, wobei eine Verbindung der Formel (I) erhalten wird, worin A die Formel (Ic), wie oben definiert, aufweist und $R^3$ (1-Piperidinyl)-methyl ist; oder

i) das Umsetzen einer Verbindung der Formel

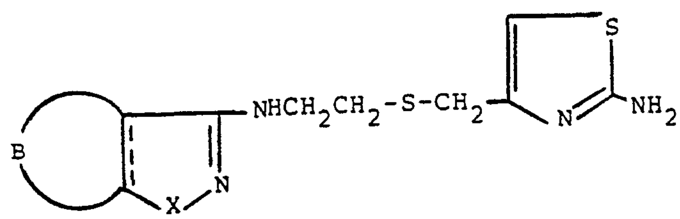

(IX)

worin die strichlierte Linie, X und B wie oben definiert sind, mit einer Verbindung, die imstande ist, —$NH_2$

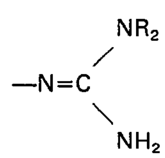

überzufuhren, wobei eine Verbindung der Formel (I) erhalten wird, worin A die Formel (Ic), wie oben definiert, aufweist; oder

j) das Umsetzen einer Verbindung der Formel

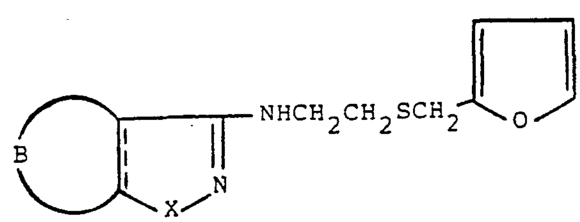

(X)

worin die strichlierte Linie, X und B wie oben definiert sind, mit Dimethylamin in Anwesenheit von Formaldehyd oder p-Formaldehyd, wobei eine Verbindung der Formel (I) erhalten wird, worin A die Formel (Ia), wie oben definiert, aufweist; oder

k) das Umsetzen einer Verbindung der Formel

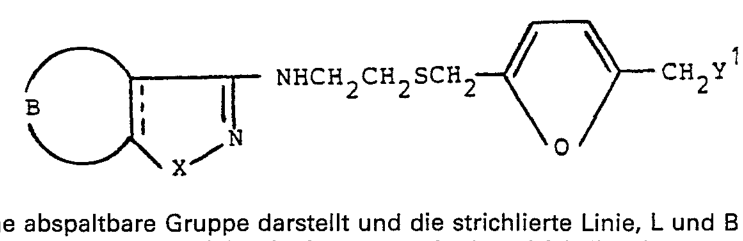

(XI)

worin $Y^1$ OH oder eine abspaltbare Gruppe darstellt und die strichlierte Linie, L und B wie oben definiert sind, mit Dimethylamin, wobei die Reaktion in Anwesenheit eines Nickelkatalysators durchgeführt wird, wenn $Y^1$ OH ist; oder

l) die Überführung einer Verbindung der Formel (I), wie oben definiert, mit einer reaktiven Substituentengruppe in eine Verbindung der Formel (I) mit einer anderen Substituentengruppe; oder

m) die Überführung einer basischen Verbindung der Formel (I), wie oben definiert, in ein pharmakologisch annehmbares Salz oder eines pharmakologisch annehmbaren Salzes in die Form der freien Base.

2. Verfahren, wie in Anspruch 1 beansprucht, worin A die Formel (Ic) oder (Ia) darstellt.

3. Verfahren, wie in Anspruch 1 oder Anspruch 2 beansprucht, worin B die Formel (Ie) darstellt.

4. Verfahren, wie in einem der Ansprüche 1 bis 3 beansprucht, worin $R^3$ Wasserstoff ist.

5. Verfahren, wie in einem der Ansprüche 1 bis 4 beansprucht, worin n 3 ist.

6. Verfahren, wie in einem der Ansprüche 1 bis 5 beansprucht, worin $R^1$ $R^2CH_2$ bedeutet, wobei $R^2$ 1-Piperidinyl ist.

7. Verfahren, wie in einem der Ansprüche 1 bis 6 beansprucht, worin X $SO_2$ ist.

8. Verfahren, wie in Anspruch 1 beansprucht, worin die hergestellte Verbindung

N-[2-[[[5-[(Dimethylamino)-methyl]-2-furanyl]-methyl]-thio]-äthyl]-thieno[3,4-d]isothiazol-3-amin-1,1-dioxid oder

[4-[[[2-[(Thieno[3,4-d]isothiazol-3-yl)-amino]-äthyl]-thio]-methyl]-2-thiazolyl]-guanidin-S′,S′-dioxid oder

N-[2-[3-(1-Piperidinylmethyl)-phenoxy]-propyl]-thieno[3,4-d]isothiazol-3-amin-1,1-dioxid oder

N-[3-[4-(1-Piperidinylmethyl)-phenoxy]-propyl]-thieno[3,4-d]isothiazol-3-amin-1,1-dioxid oder ein pharmakologisch annehmbares Salz hievon ist.

9. Verfahren zum Herstellen einer pharmazeutischen Zusammensetzung, dadurch gekennzeichnet, daß eine Verbindung der Formel (I), wie in einem der Ansprüche 1 bis 8 definiert, oder ein pharmakologisch annehmbares Salz hievon mit einem pharmazeutischen Träger gemischt und in eine für therapeutische Verabreichung geeignete Form gebracht wird.

10. Verfahren, wie in Anspruch 9 beansprucht, worin die Verbindung der Formel (I) nach einem Verfahren, wie in einem der Ansprüche 1 bis 8 beansprucht, hergestellt ist.

**Revendications pour les Etats contractants: BE CH DE FR GB IT LI LU NL et SE**

Composé de formule générale

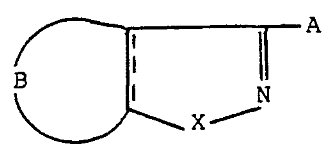

(I)

dans laquelle B est un groupement de formule:

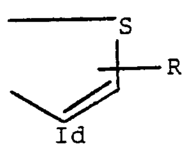
Id

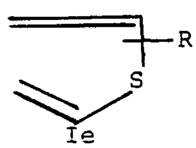
Ie

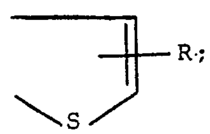
If

le segment en traits interrompus représente une liaison qui existe lorsque B répond à la formule Id ou If et qui est absente lorsque B répond à la formule Ie, R est l'hydrogène, un radical monohalogéno ou dihalogéno, amino, nitro, cyano, hydroxy, trifluorométhyle, mercapto, alkyle inférieur, alkoxy inférieur, alcanoyle de 2 à 5 atomes de carbone, cycloalkyle de 5 à 7 atomes de carbone, carboxy, alkoxycarbonyle, amino portant un ou deux substituants alkyle inférieurs, alcanoylamino de 2 à 5 atomes de carbone, alkylthio inférieur, alkylsulfonyle inférieur, sulfamoyle, sulfamoyle à substituant alkyle inférieur, phényle, ou phényle substitué avec un radical halogéno, alkyle inférieur, alkoxy inférieur, trifluorométhyle, hydroxy, amino, cyano ou nitro; X représente $SO_2$, SO, S ou C=O, et A est une amine de formule:

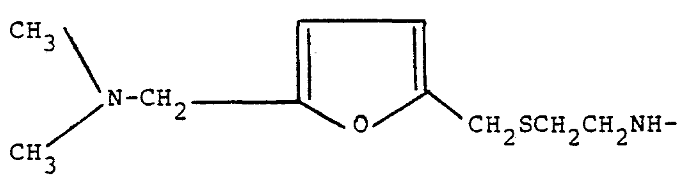

(Ia),

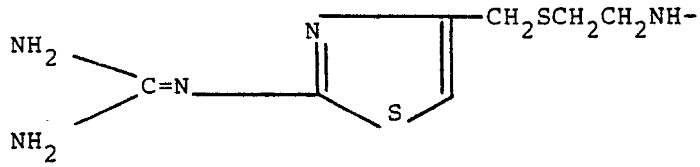

(Ib)

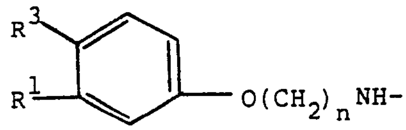

(Ic)

dans laquelle n a une valeur de 1 à 4; $R^1$ représente l'hydrogène ou un groupe $R^2CH_2$ dans lequel $R^2$ est un radical mono- ou di(alkyle inférieur)amino, mono- ou di-N-(alkyle inférieur)aminoalkyle inférieur, (2-furyl)méthylamino, benzylamino, cycloalkylamino de 5 à 7 atomes de carbone, 1-pyrrolidinyle, 1-pipéridinyle, 1-hexahydroazépinyle, 1-octahydroazocinyle, 3-thioazolidinyle, 4-morpholinyle ou 4-thiomorpholinyle; $R^3$ désigne l'hydrogène ou un groupe (1-pipéridinyl)méthyle sous réserve que lorsque $R^3$ est un group (1-pipéridinyl)méthyle, $R^1$ est un atome d'hydrogène, le terme "inférieur" utilisé ici pour qualifier un groupe "alkyle" ou "alkoxy" signifiant que ces groupes contiennent 1 à 6 atomes de carbone; et ses sels pharmacologiquement acceptables.

2. Composé suivant la revendication 1, dans lequel A représente la formule Ic ou Ia.

3. Composé suivant la revendication 1 ou la revendication 2, dans lequel B représente la formule Ie.

4. Composé suivant l'une quelconque des revendications 1 à 3, dans lequel $R^3$ est l'hydrogène.

5. Composé suivant l'une quelconque des revendications 1 à 4, dans lequel n est égal à 3.

6. Composé suivant l'une quelconque des revendications 1 à 5, dans lequel $R^1$ est un groupe $R^2CH_2$ où $R^2$ représente un groupe 1-pipéridinyle.

7. Composé suivant l'une quelconque des revendications 1 à 6, dans lequel X représente $SO_2$.

8. Composé de formule I, qui est

le 1,1-dioxyde de N-[2-[[[5-[(diméthylamino)méthyl]-2-furannyl]méthyl]-thio]éthyl]thiéno[3,4-d]isothiazole-3-amine; ou

le S',S'-dioxyde de [4-[[[2-[(thiéno[3,4-*d*]isothiazole-3-yl)amino]éthyl]-thio]méthyl]-2-thiazolyl]-guanidine; ou

le 1,1-dioxyde de *N*-[2-[3-(1-pipéridinylméthyl)phénoxy]propyl]thiéno-[3,4-d]isothiazole-3-amine; ou

le 1,1-dioxyde de *N*-[3-[4-(1-pipéridinylméthyl)phénoxy]propyl]thiéno-[3,4-*d*]isothiazole-3-amine; ou un sel pharmacologiquement acceptable de ce composé.

9. Composé suivant l'une quelconque des revendications 1 à 8, sous la forme d'un sel avec un acide choisi entre less acides chlorhydrique, bromhydrique, sulfurique, nitrique, phosphorique, acétique, maléique, fumarique, citrique et méthanesulfonique.

10. Procédé de préparation d'un composé de formule I tel que défini dans la revendication 1, qui consiste:

a) à faire réagir un composé de formule

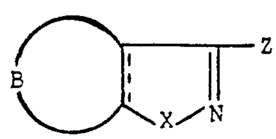

(II)

dans laquelle le segment, en traits interrompus, X et B sont tels que définis dans la revendication 1 et Z représente un groupe $SR^4$ dans lequel $R^4$ est un radical alkyle ou aralkyle, avec une amine de formule H—A dans laquelle A est tel que défini dans la revendication 1 pour obtenir un composé de formule I, ou bien

b) à faire réagir un composé de formule

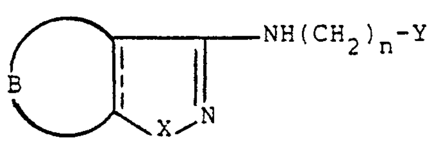

(III)

dans laquelle le segment en traits interrompus, X, B et n sont tels que définis dans la revendication 1 et Y est un groupe OH ou L, ce dernier représentant un groupe partant ou un groupe équivalent remplaçable, avec un composé de formule:

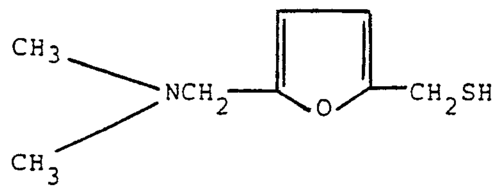

(IVa)

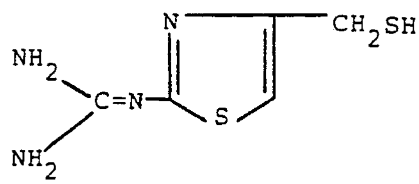

(IVb)

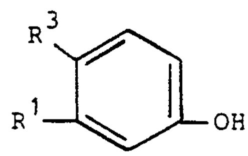

(IVc)

où $R^1$ et $R^3$ sont tels que définis dans la revendication 1, sous réserve que (i) lorsqu'un composé de formule IVa ou IVb est utilisé, n soit égal à 2 dans le composé de formule III et que (ii) lorsqu'un composé de formule IVc est utilisé, Y représente alors L; ou bien

c) à faire réagir un composé de formule

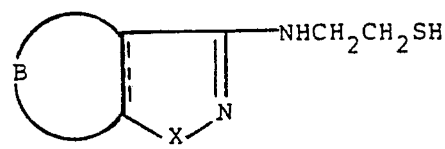

(V)

dans laquelle le segment en traits interrompus, B et X sont tels que définis dans la revendication 1, acec un composé de formule:

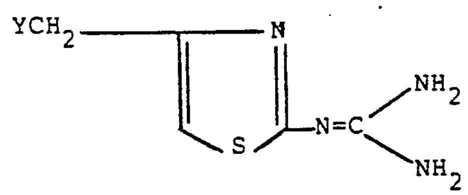

(VIa)

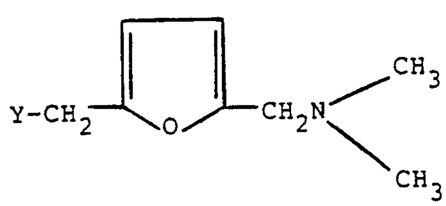

(VIb)

dans laquelle Y représente OH ou L, ce dernier étant un groupe partant, pour former un composé de formule I dans laquelle A répond à formule A répond à la formule Ia ou Ib telle que définie dans la revendication 1; ou bien

d) à faire réagir un aldéhyde de formule

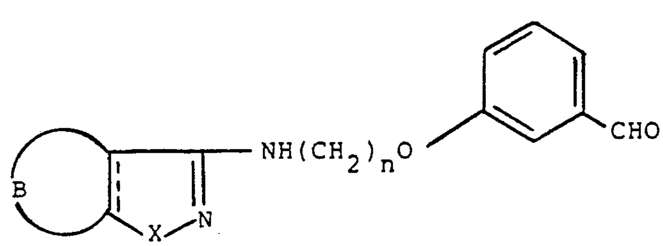

(VIIa)

avec une amine appropriée de formule

HR²    (VIIb)

formules dans lesquelles le segment en traits interrompus, n, B, X et $R^2$ sont tels que définis dans la revendication 1, dans des conditions d'amination par voie de réduction pour obtenir un composé correspondant de formule I dans laquelle $R^1$ est un groupe $R^2CH_2$— et $R^3$ est un atome d'hydrogène; ou bien

e) à faire réagir un aldéhyde de formule

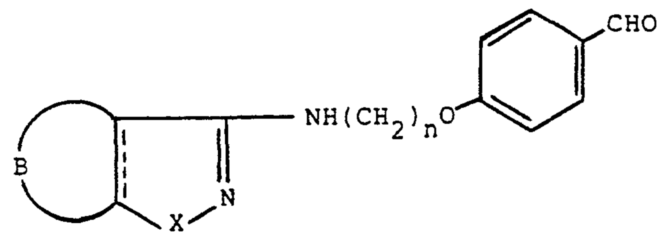

(VIIc)

dans laquelle le segment en traits interrompus, n, B et X sont tels que définis dans la revendication 1 avec la pipéridine dans des conditions d'amination par voie de réduction pour obtenir un composé de formule I dans laquelle $R^3$ est un groupe (1-pipéridinyl)méthyle; ou bien

f) à faire réagir un aldéhyde de formule

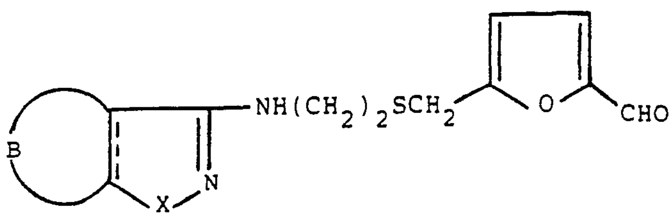

(VIId)

dans laquelle le segment en traits interrompus, B et X sont tels que définis dans la revendication 1, avec la diméthylamine dans des conditions d'amination par voie de réduction pour obtenir un composé de formule I dans laquelle A répond à la formule Ia telle que définie dans la revendication 1; ou bien

g) à faire réagir un composé de formule

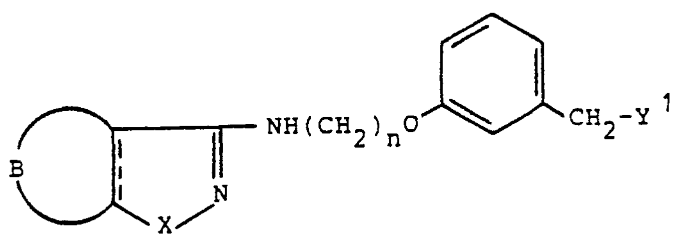

(VIII)

dans laquelle le segment en traits interrompus, n, X et B sont tels que définis dans la revendication 1 et $Y^1$ est un groupe OH ou un groupe L qui est un groupe partant, avec une amine de formule (VIIb)

$HR^2$

dans laquelle $R^2$ est tel que défini dans la revendication 1, la réaction étant conduite en présence d'un catalyseur au nickel lorsque Y représente OH, pour former un composé de formule I dans laquelle A répond à la formule Ic et $R^1$ est un groupe $R^1$—$CH_2$—; ou bien

h) à faire réagir un composé de formule

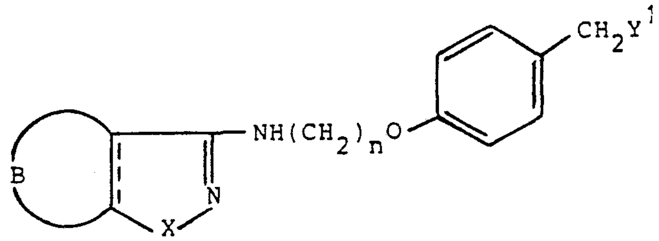

(VIIIa)

dans laquelle le segment en traits interrompus, $Y^1$, n, X et B sont tels que définis dans la revendication 1, acec la pipéridine, la réaction étant conduite en présence d'un catalyseur au nickel lorsque $Y^1$ est un groupe

OH, pour former un composé de formule I dans laquelle A répond à la formule Ic telle que définie dans la revendication 1 et $R^3$ est un groupe (1-pipéridinyl)méthyle; ou bien

i) à faire réagir un composé de formule

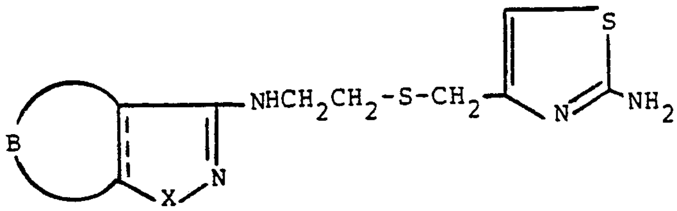

(IX)

dans laquelle le segment en traits interrompus, X et B sont tels que définis dans la revendication 1, avec un composé capable de convertir $—NH_2$ en un groupe

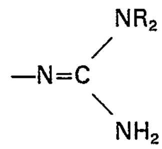

pour former un composé de formule I dans laquelle A répond à la formule Ic, telle que définie dans la revendication 1, ou bien

j) à faire réagir un composé de formule

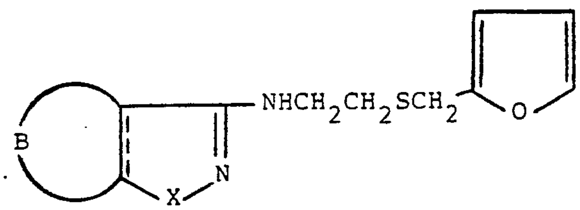

(X)

dans laquelle le segment en traits interrompus, X et B sont tels que définis dans la revendication 1, avec la diméthylamine en présence de formaldéhyde ou de paraformaldéhyde pour obtenir un composé de formule I dans laquelle A répond à la formule Ia telle que définie dans la revendication 1, ou bien

k) à faire réagir un composé de formule

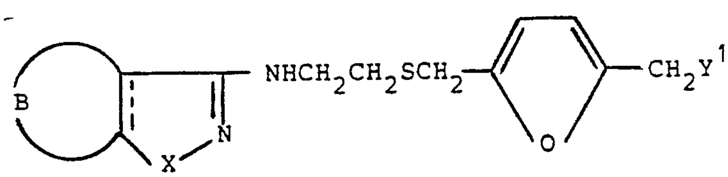

(XI)

dans laquelle $Y^1$ est un groupe OH ou un groupe partant et le segment en traits interrompus, X et B sont tels que définis dans la revendication 1, avec la diméthylamine; la réaction étant conduite en présence d'un catalyseur au nickel lorsque $Y^1$ est un groupe OH; ou bien

l) à convertir un composé de formule I telle que définie dans la revendication 1 portant un groupe substituant réactif, en un composé de formule I portant un groupe substituant différent; ou bien

m) à convertir un composé de base de formule I telle que définie dans la revendication 1 en un sel pharmacologiquement acceptable ou à convertir un sel pharmacologiquement acceptable en la forme base libre.

11. Composé de formule I ou un sel pharmacologiquement acceptable de ce composé tel que défini dans l'une quelconque des revendications 1 à 9, destiné à être utilisé comme substance pharmaceutique.

12. Composition pharmaceutique comprenant un composé de formule I ou un sel pharmacologiquement acceptable tel que défini dans l'une quelconque des revendications 1 à 9 et un support acceptable du point de vue pharmaceutique.

**Revendications pour l'Etat contractant: AT**

Procédé de préparation d'un composé de formule

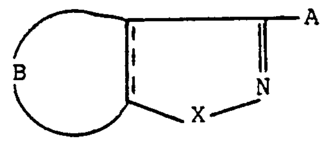

(I)

dans laquelle B est un groupement de formule:

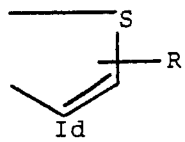

Id

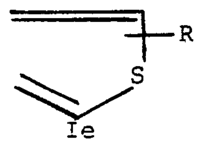

Ie

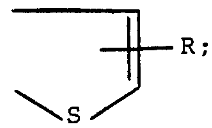

If

le segment en traits interrompus représente une liaison qui existe lorsque B répond à la formule Id ou If et qui est absente lorsque B répond à la formule Ie, R est l'hydrogène, un radical monohalogéno ou dihalogéno, amino, nitro, cyano, hydroxy, trifluorométhyle, mercapto, alkyle inférieur, alkoxy inférieur, alcanoyle de 2 à 5 atomes de carbone, cycloalkyle de 5 à 7 atomes de carbone, carboxy, alkoxycarbonyle, amino portant un ou deux substituants alkyle inférieurs, alcanoylamino de 2 à 5 atomes de carbone, alkylthio inférieur, alkylsulfonyle inférieur, sulfamoyle, sulfamoyle à substituant alkyle inférieur, phényle, ou phényle substitué avec un radical halogéno, alkyle inférieur, alkoxy inférieur, trifluorométhyle, hydroxy, amino, cyano ou nitro; X représente $SO_2$, SO, S ou C=O, et A est une amine de formule:

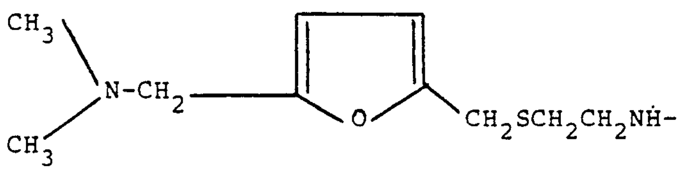

(Ia),

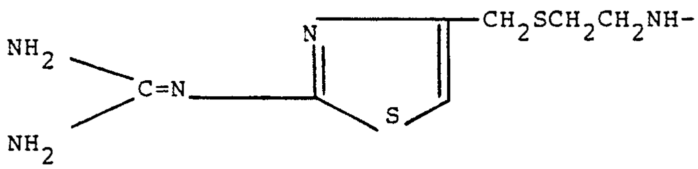

(Ib)

or

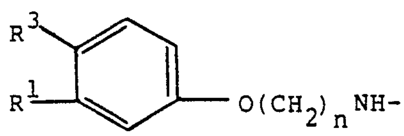

(Ic)

dans laquelle n a une valeur de 1 à 4; $R^1$ représente l'hydrogène ou un groupe $R^2CH_2$ dans lequel $R^2$ est un radical mono- ou di(alkyle inférieur)amino, mono- ou di-N-(alkyle inférieur)aminoalkyle inférieur, (2-furyl)méthylamino, benzylamino, cycloalkylamino de 5 à 7 atomes de carbone, 1-pyrrolidinyle, 1-pipéridinyle, 1-hexahydroazépinyle, 1-octahydroazocinyle, 3-thioazolidinyle, 4-morpholinyle ou 4-thiomorpholinyle; $R^3$ désigne l'hydrogène ou un groupe (1-pipéridinyl)méthyle sous réserve que lorsque $R^3$ est un group (1-pipéridinyl)méthyle, $R^1$ est un atome d'hydrogène, le terme "inférieur" utilisé ici pour qualifier un groupe "alkyle" ou "alkoxy" signifiant que ces groupes contiennent 1 à 6 atomes de carbone; et de ses sels pharmacologiquement acceptables, qui consiste

a) à faire réagir un composé de formule

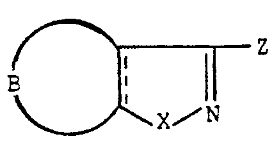

(II)

dans laquelle le segment en traits interrompus, X et B sont tels que définis ci-dessus et Z représente un

groupe $SR^4$ dans lequel $R^4$ est un radical alkyle ou aralkyle, avec une amine de formule H—A dans laquelle A est tel que défini ci-dessus pour obtenir un composé de formule I, ou bien

b) à faire réagir un composé de formule

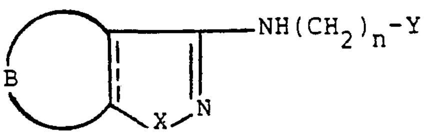

(III)

dans laquelle le segment en traits interrompus, X, B et n sont tels que définis ci-dessus et Y est un groupe OH ou L, ce dernier représentant un groupe partant ou un groupe équivalent remplaçable avec un composé de formule:

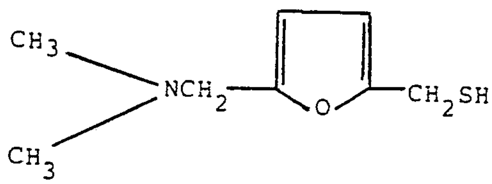

(IVa)

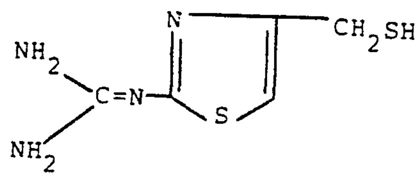

(IVb)

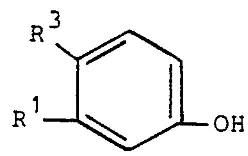

(IVc)

où $R^1$ et $R^3$ sont tels que définis ci-dessus, sous réserve que (i) lorsqu'un composé de formule IVa ou IVb est utilisé, n soit égal à 2 dans le composé de formule III et que (ii) lorsqu'un composé de formule IVc est utilisé, Y représente alors L; ou bien

c) à faire réagir un composé de formule

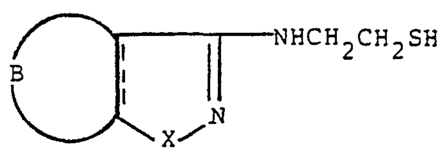

(V)

dans laquelle le segment en traits interrompus, B et X sont tels que définis ci-dessus, acec un composé de formule:

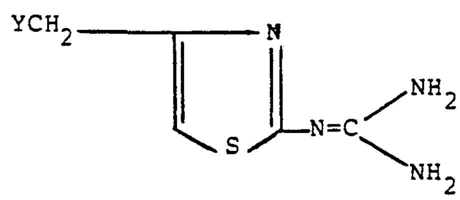

(VIa)

ou

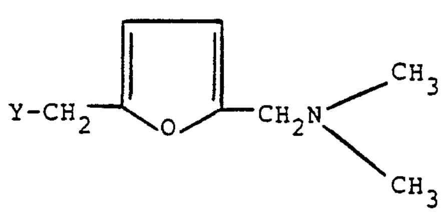

(VIb)

dans laquelle Y représente OH ou L, ce dernier étant un groupe partant, pour former un composé de formule I dans laquelle A répond à la formule Ia ou Ib telle que définie ci-dessus; ou bien

d) à faire réagir un aldéhyde de formule

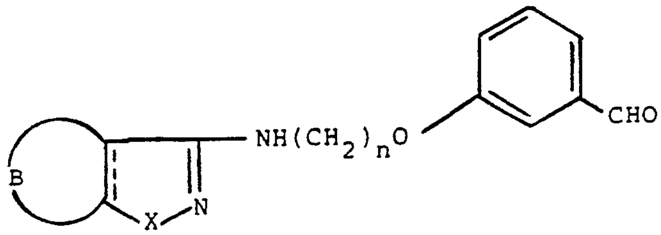

(VIIa)

avec une amine appropriée de formule

$HR^2$ (VIIb)

formules dans lesquelles le segment en traits interrompus, n, B, X et $R^2$ sont tels que définis ci-dessus, dans des conditions d'amination par voie de réduction pour obtenir un composé correspondant de formule I dans laquelle $R^1$ est un groupe $R^2CH_2$— et $R^3$ est un atome d'hydrogène; ou bien

e) à faire réagir un aldéhyde de formule

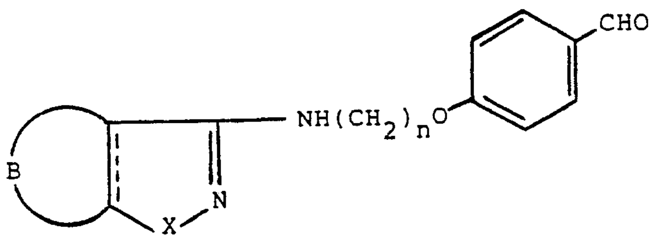

(VIIc)

dans laquelle le segment en traits interrompus, n, B et X sont tels que définis ci-dessus avec la pipéridine dans des conditions d'amination par voie de réduction pour obtenir un composé de formule I dans laquelle $R^3$ est un groupe (1-pipéridinyl)méthyle; ou bien

f) à faire réagir un aldéhyde de formule

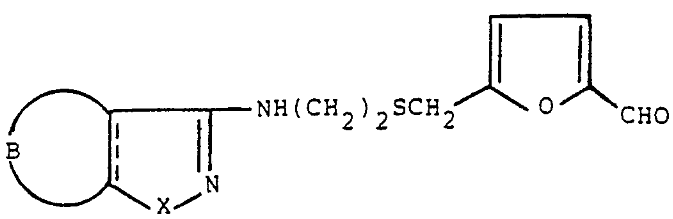

(VIId)

dans laquelle le segment en traits interrompus, B et X sont tels que définis ci-dessus, avec la diméthylamine dans des conditions d'amination par voie de réduction pour obtenir un composé de formule I dans laquelle A répond à la formule Ia telle que définie ci-dessus; ou bien

g) à faire réagir un composé de formule

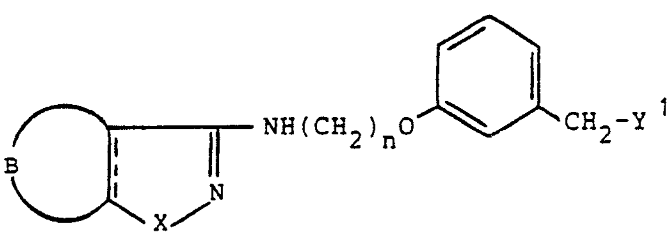

(VIII)

dans laquelle le segment en traits interrompus, n, X et B sont tels que définis ci-dessus et $Y^1$ est un groupe OH ou un groupe L qui est un groupe partant, avec une amine de formule (VIIb)

$HR^2$

dans laquelle $R^2$ est tel que défini ci-dessus, la réaction étant conduite en présence d'un catalyseur au nickel lorsque $Y^1$ représente OH, pour former un composé de formule I dans laquelle A répond à la formule Ic et $R^1$ est un groupe $R^1$—$CH_2$—; ou bien

h) à faire réagir un composé de formule

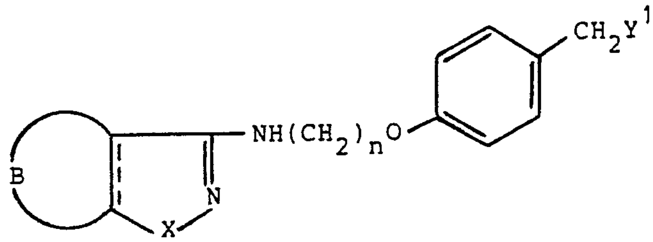

(VIIIa)

dans laquelle le segment en traits interrompus, $Y^1$, n, X et B sont tels que définis ci-dessus, acec la pipéridine, la réaction étant conduite en présence d'un catalyseur au nickel lorsque $Y^1$ est un groupe OH, pour former un composé de formule I dans laquelle A répond à la formule Ic telle que définie ci-dessus et $R^3$ est un groupe (1-pipéridinyl)méthyle; ou bien

i) à faire réagir un composé de formule

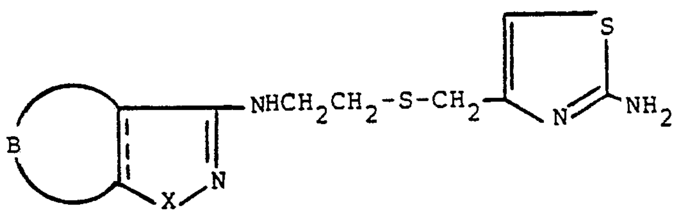

(IX)

dans laquelle le segment en traits interrompus, X et B sont tels que définis ci-dessus, avec un composé capable de convertir —$NH_2$ en un groupe

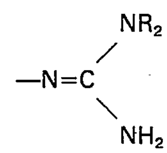

pour former un composé de formule I dans laquelle A répond à la formule Ic, telle que définie ci-dessus, ou bien

j) à faire réagir un composé de formule

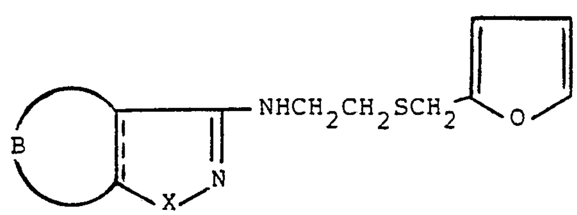

(X)

dans laquelle le segment en traits interrompus, X et B sont tels que définis ci-dessus, avec la diméthylamine en présence de formaldéhyde ou de paraformaldéhyde pour obtenir un composé de formule I dans laquelle A répond à la formule Ia telle que définie ci-dessus, ou bien

k) à faire réagir un composé de formule

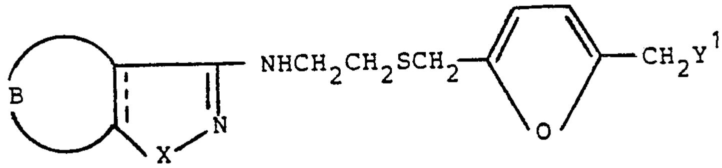

(XI)

dans laquelle $Y^1$ est un groupe OH ou un groupe partant et le segment en traits interrompus, X et B sont tels que définis ci-dessus, avec la diméthylamine; la réaction étant conduite en présence d'un catalyseur au nickel lorsque $Y^1$ est un groupe OH; ou bien

l) à convertir un composé de formule I telle que définie ci-dessus portant un groupe substituant réactif, en un composé de formule I portant un groupe substituant différent; ou bien

m) à convertir un composé de base de formule I telle que définie ci-dessus en un sel pharmacologiquement acceptable ou à convertir un sel pharmacologiquement acceptable en la forme base libre.

2. Procédé suivant la revendication 1, dans lequel A représente la formule Ic ou la.

3. Procédé suivant la revendication 1 ou la revendication 2, dans lequel B représente la formule le.

4. Procédé suivant l'une quelconque des revendications 1 à 3, dans lequel $R^3$ est l'hydrogène.

5. Procédé suivant l'une quelconque des revendications 1 à 4, dans lequel n est égal à 3.

6. Procédé suivant l'une quelconque des revendications 1 à 5, dans lequel $R^1$ représente un groupe $R^2CH_2$ où $R^2$ est un groupe 1-pipéridinyle.

7. Procédé suivant l'une quelconque des revendications 1 à 6, dans lequel X est un groupe $SO_2$.

8. Procédé suivant la revendication 1, dans lequel le composé préparé est

le 1,1-dioxyde de N-[2-[[[5-[(diméthylamino)méthyl]-2-furannyl]méthyl]-thio]éthyl]thiéno[3,4-d]-isothiazole-3-amine, ou

le S',S'-dioxyde de [4-[[[2-[(thiéno[3,4-*d*]isothiazole-3-yl)amino]éthyl]-thio]méthyl]-2-thiazolyl]guanidine, ou

le 1,1-dioxyde de *N*-[2-[3-(1-pipéridinylméthyl)phénoxy]propyl]thiéno-[3,4-d]isothiazole-3-amine, ou

le 1,1-dioxyde de *N*-[3-[4-(1-pipéridinylméthyl)phénoxy]propyl]thiéno-[3,4-*d*]isothiazole-3-amine; ou un sel pharmacologiquement acceptable de ce composé.

9. Procédé de préparation d'une composition pharmaceutique, caractérisé en ce qu'un composé de formule I tel que défini selon l'une quelconque des revendications 1 à 8 ou un sel pharmacologiquement acceptable de ce composé est mélangé avec un support pharmaceutique et le mélange est mis sous une forme qui convient à l'administration thérapeutique.

10. Procédé suivant la revendication 9, dans lequel le composé de formule I est préparé par un procédé suivant l'une quelconque des revendications 1 à 8.